(19) Europäisches Patentamt
European Patent Office
Office européen des brevets

(11) Veröffentlichungsnummer: **0 042 980**
**A2**

(12) **EUROPÄISCHE PATENTANMELDUNG**

(21) Anmeldenummer: **81104157.3**

(22) Anmeldetag: **01.06.81**

(51) Int. Cl.³: **C 07 D 249/08**
C 07 D 233/60, A 01 N 43/64
A 01 N 43/50
//C07C49/255, C07C143/68,
C07C49/17, C07C149/34,
C07C149/16

(30) Priorität: **07.06.80 DE 3021551**

(43) Veröffentlichungstag der Anmeldung:
**06.01.82 Patentblatt 82/1**

(84) Benannte Vertragsstaaten:
**BE CH DE FR GB IT LI NL**

(71) Anmelder: **BAYER AG**
**Zentralbereich Patente, Marken und Lizenzen**
**D-5090 Leverkusen 1, Bayerwerk(DE)**

(72) Erfinder: **Krämer, Wolfgang, Dr.**
**Am Eckbusch 39/45**
**D-5600 Wuppertal 1(DE)**

(72) Erfinder: **Elbe, Hans-Ludwig, Dr.**
**Dasnoeckel 59**
**D-5600 Wuppertal 11(DE)**

(72) Erfinder: **Büchel, Karl Heinz, Prof.Dr.**
**Dabringhausener Strasse 42**
**D-5093 Burscheid(DE)**

(72) Erfinder: **Brandes, Wilhelm, Dr.**
**Eichendorffstrasse 3**
**D-5653 Leichlingen(DE)**

(72) Erfinder: **Frohberger, Paul-Ernst, Dr.**
**Willi-Baumeister-Strasse 5**
**D-5090 Leverkusen(DE)**

(54) **4-Substituierte 1-Azolyl-l-phenoxy-3,3-dimethyl-butan-2-one und -ole, Verfahren zu ihrer Herstellung und ihre Verwendung als Fungizide.**

(57) Die Erfindung betrifft neue 4-substituierte 1-Azolyl-1-phenoxy-3,3-dimethyl-butan-2-one und -ole, ein Verfahren zu ihrer Herstellung und ihre Verwendung als Fungizide.

Die Verbindungen der allgemeinen Formel

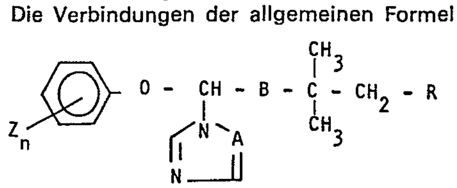

(I)

in welcher A, B, R, Z und n die in der Beschreibung angebene Bedeutung besitzen, werden erhalten, wenn man Halogenetherketone mit Azolen gegebenenfalls in Gegenwart eines Verdünnungsmittels und in Gegenwart eines Säurebindemittels umsetzt, und gegebenenfalls noch die erhaltenen Keto-Derivate nach bekannten Methoden in üblicher Weise reduziert, und gegebenenfalls noch die erhaltenen Thio-Derivate nach gekannten Methoden in üblicher Weise oxidiert.

Die Wirkstoffe sind für den Gebrauch als Pflanzenschutzmittel geeignet und können mit besonders gutem Erfolg zur Bekämpfung von Getreidekrankheiten und von Venturia-Arten, von Uromyces-Arten sowie von Erysiphe-Arten eingesetzt werden.

EP 0 042 980 A2

Croydon Printing Company Ltd.

BAYER AKTIENGESELLSCHAFT
Zentralbereich
Patente, Marken und Lizenzen

5090 Leverkusen, Bayerwerk
Slr/W
Ia

# 4-substituierte 1-Azolyl-1-phenoxy-3,3-dimethyl-butan-2-one und -ole, Verfahren zu ihrer Herstellung und ihre Verwendung als Fungizide

Die vorliegende Erfindung betrifft neue 4-substituierte 1-Azolyl-1-phenoxy-3,3-dimethyl-butan-2-one und -ole, ein Verfahren zu ihrer Herstellung und ihre Verwendung als Fungizide.

Es ist bereits bekannt geworden, daß allgemein Azolyl-alkyl-Derivate, wie beispielsweise 1-Hydroxyethyl-triazol-Derivate und insbesondere Triazolyl-und Imidazolylether-ketone und -carbinole gute fungizide Eigenschaften aufweisen (vergleiche DE-PS 22 01 063 [LeA 14 118], DE-OS 23 24 010 [LeA 14 971], DE-OS 24 31 407 [LeA 15 735], DE-OS 26 32 603 [LeA 17 273], DE-OS 26 32 602 [LeA 17 274] und DE-OS 26 35 666 [LeA 17 324]).
Die Wirkung dieser Verbindungen ist jedoch, insbesondere bei niedrigen Aufwandmengen und -konzentrationen, nicht immer ganz befriedigend.

Es wurden neue 4-substituierte 1-Azolyl-1-phenoxy-3,3-dimethyl-butan-2-one und -ole der allgemeinen Formel

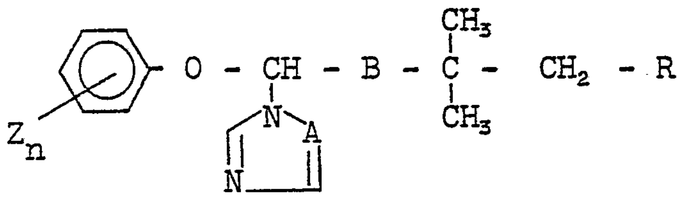

(I)

in welcher

A für ein Stickstoffatom oder die CH-Gruppe steht,

B für die Ketogruppe oder die CH(OH)-Gruppe steht,

R für die Gruppierung X-$R^1$ oder Cyano steht, wobei

$R^1$ für Alkyl, gegebenenfalls substituiertes Aryl oder gegebenenfalls substituiertes Aralkyl steht, und

X für Sauerstoff, Schwefel, die SO- oder $SO_2$-Gruppe steht,

Z für Halogen, Alkyl, Halogenalkyl, Alkoxy, Alkylthio, Alkylsulfonyl, Nitro, Cyano, Alkoxycarbonyl oder gegebenenfalls substituiertes Phenyl steht und

n für 0, 1, 2 oder 3 steht,

sowie deren physiologisch verträglichen Säureadditions-Salze und Metallsalz-Komplexe gefunden.

Diejenigen Verbindungen der Formel (I), in welchen B für die CH(OH)-Gruppe steht, besitzen zwei asymmetrische Kohlenstoffatome; sie können deshalb in zwei geometrischen Isomeren (threo- und erythro- Form) vorliegen, die in unterschiedlichen Mengenverhältnissen anfallen können. In

beiden Fällen liegen sie als optische Isomeren vor. Sämtliche Isomeren werden erfindungsgemäß beansprucht.

Weiterhin wurde gefunden, daß man die 4-substituierten 1-Azolyl-1-phenoxy-3,3-dimethyl-butan-2-one und -ole der Formel (I) erhält, wenn man Halogenetherketone der Formel

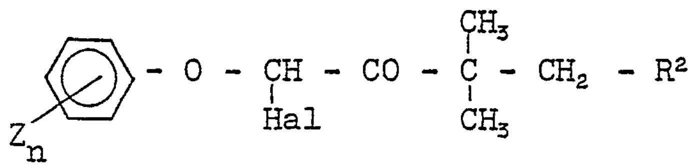 (II)

in welcher

Z und n die oben angegebene Bedeutung haben,
Hal für Halogen, vorzugsweise Chlor oder Brom steht und
$R^2$ für $X^1$-$R^1$ oder Cyano steht, wobei
$X^1$ für Sauerstoff oder Schwefel steht und
$R^1$ die oben angegebene Bedeutung hat,

mit Azolen der Formel

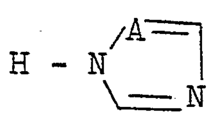 (III)

in welcher

A die oben angegebene Bedeutung hat,

gegebenenfalls in Gegenwart eines Verdünnungsmittels und in Gegenwart eines Säurebindemittels umsetzt, und

gegebenenfalls noch die erhaltenen Keto-Derivate der Formel

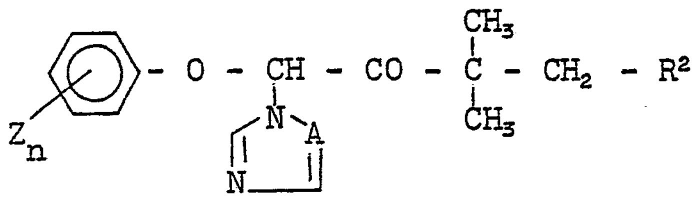

(Ia)

in welcher

A, $R^2$, Z und n die oben angegebene Bedeutung haben,

nach bekannten Methoden in üblicher Weise reduziert, und gegebenenfalls noch die erhaltenen Thio-Derivate der Formel

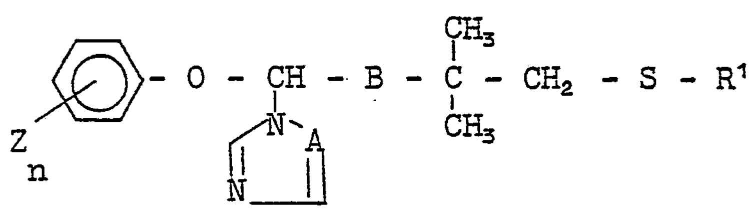

(Ib)

in welcher
A, B, Z, $R^1$ und n die oben angegebene Bedeutung haben,

nach bekannten Methoden in üblicher Weise oxidiert.

An die so erhaltenen Verbindungen der Formel (I) können gegebenenfalls anschließend eine Säure oder ein Metallsalz addiert werden. In manchen Fällen erweist sich als vorteilhaft, die Verbindungen der Formel (I) über ihre Salze in reiner Form zu erhalten.

Die neuen 4-substituierten 1-Azolyl-1-phenoxy-3,3-dimethyl-butan-2-one und -ole weisen starke fungizide Eigenschaften auf. Dabei zeigen überraschenderweise die erfindungsgemäßen Verbindungen eine erheblich höhere Wirkung als die aus dem Stand der Technik bekannten Azolyl-alkyl-Derivate, die chemisch und/oder wirkungsmäßig naheliegende Verbindungen sind. Die erfindungsgemäßen Stoffe stellen somit eine Bereicherung der Technik dar.

Die erfindungsgemäßen 4-substituierten 1-Azolyl-1-phenoxy-3,3-dimethyl-butan-2-one und -ole sind durch die Formel (I) allgemein definiert. In dieser Formel steht R vorzugsweise für die Gruppierung $X-R^1$ sowie für Cyano.

$R^1$ steht vorzugsweise für geradkettiges oder verzweigtes Alkyl mit 1 bis 6 Kohlenstoffatomen sowie für gegebenenfalls substituiertes Aryl mit 6 bis 10 Kohlenstoffatomen und gegebenenfalls substituiertes Aralkyl mit 6 bis 10 Kohlenstoffatomen im Arylteil und 1 bis 2 Kohlenstoffatomen im Alkylteil, wobei als Substituenten vorzugsweise genannt seien: Halogen, Cyano, Nitro, Alkoxycarbonyl mit 1 bis 4 Kohlenstoffatomen im Alkylteil, Alkyl und Alkoxy mit jeweils 1 bis 4 Kohlenstoffatomen, Dialkylamino mit 1 bis 2 Kohlenstoffatomen in jedem Alkylteil, sowie gegebenenfalls durch Halogen substituiertes Phenyl.

Z steht vorzugsweise für Halogen, geradkettiges oder verzweigtes Alkyl und Alkoxy mit jeweils 1 bis 4 Kohlenstoffatomen, Halogenalkyl mit 1 bis 2 Kohlenstoffatomen und 1 bis 5 gleichen oder verschiedenen Halogenatomen, wie insbesondere Fluor- und Chloratomen, Alkylthio mit 1 bis 4 Kohlenstoffatomen, Alkylsulfonyl mit 1 bis 4 Kohlenstoffatomen, Nitro, Cyano, Alkoxycarbonyl mit 1 bis 4 Kohlenstoffatomen im Alkylteil, sowie für gegebenenfalls durch Halogen substituiertes Phenyl.

A, B, X und der Index n haben vorzugsweise die in der Erfindungsdefinition angegebene Bedeutung.

Besonders bevorzugt sind diejenigen Verbindungen der Formel (I), in denen R für die Gruppierung $X-R^1$ oder Cyano steht; $R^1$ für geradkettiges oder verzweigtes Alkyl mit 1 bis 4 Kohlenstoffatomen oder für gegebenenfalls substituiertes Phenyl und Benzyl steht, wobei als Substituenten genannt seien: Fluor, Chlor, Brom, Cyano, Nitro, Methoxycarbonyl, Methyl, Ethyl, Isopropyl, tert.-Butyl, Methoxy, Dimethylamino sowie gegebenenfalls durch Fluor und Chlor substituiertes Phenyl; Z für Fluor, Chlor, Brom, Jod, Methyl, Ethyl, Isopropyl, tert.-Butyl, Methoxy, Nitro, Cyano, Methoxycarbonyl, Ethoxycarbonyl, Methylthio, Methylsulfonyl, Trifluormethyl sowie gegebenenfalls durch Fluor und Chlor substituiertes Phenyl steht; der Index n für 0, 1 oder 2 steht, und A, B sowie X die in der Erfindungsdefinition angegebene Bedeutung haben.

Im einzelnen seien außer den bei den Herstellungsbeispielen gananmten Verbindungen die folgenden Verbindungen der allgemeinen Formel (I) genannt:

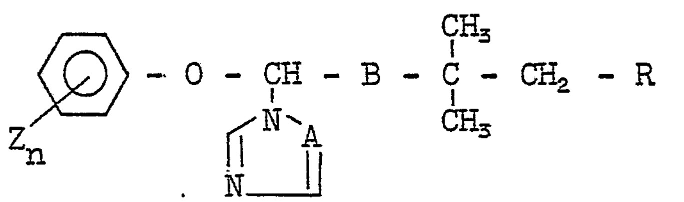

(I)

| $Z_n$ | A | B | R |
|---|---|---|---|
| 4-Cl | CH | CO | 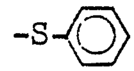 |
| 4-Cl | CH | CO | 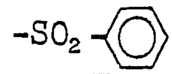 |
| 4-Cl | CH | CO | 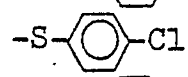 |
| 4-Cl | CH | CO | 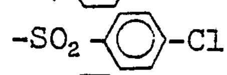 |
| 4-Cl | CH | CH(OH) | 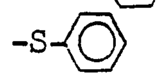 |
| 4-Cl | CH | CH(OH) | 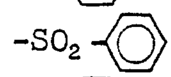 |
| 4-Cl | CH | CH(OH) | 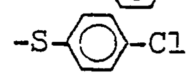 |
| 4-Cl | CH | CH(OH) | 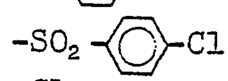 |
| 4-CN | N | CO | 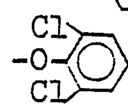 |
| 4-CN | CH | CO | 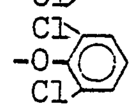 |
| 4-CN | N | CH(OH) | 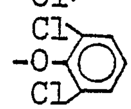 |
| 4-CN | CH | CH(OH) | 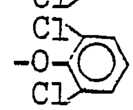 |
| $4-COOCH_3$ | N | CO | 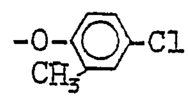 |
| $4-COOCH_3$ | CH | CO | 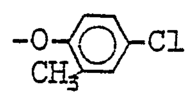 |
| $4-COOCH_3$ | N | CH(OH) | 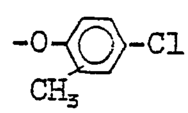 |
| $4-COOCH_3$ | CH | CH(OH) | 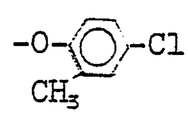 |
| 4-Cl | N | CO | $-O-C_3H_7$ |
| 4-Cl | N | CH(OH) | $-O-C_3H_7$ |
| 4-Cl | CH | CO | $-O-C_3H_7$ |
| 4-Cl | CH | CH(OH) | $-O-C_3H_7$ |

| $Z_n$ | A | B | R |
|---|---|---|---|
| 4-Cl | N | CO | $-O-C(CH_3)_3$ |
| 4-Cl | N | CH(OH) | $-O-C(CH_3)_3$ |
| 4-Cl | CH | CO | $-O-C(CH_3)_3$ |
| 4-Cl | CH | CH(OH) | $-O-C(CH_3)_3$ |
| 2,4-$Cl_2$ | N | CO | 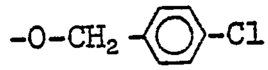 |
| 2,4-$Cl_2$ | N | CH(OH) | 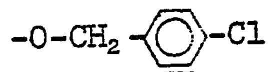 |
| 2,4-$Cl_2$ | CH | CO | 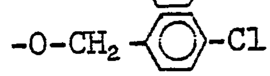 |
| 2,4-$Cl_2$ | CH | CH(OH) | 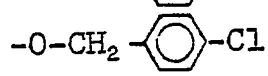 |
| 4-Cl,2-$SCH_3$ | N | CO | 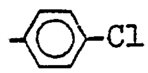 |
| 4-Cl,2-$SCH_3$ | CH | CO | 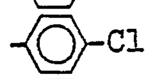 |
| 4-Cl,2-$SCH_3$ | N | CH(OH) | 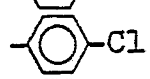 |
| 4-Cl,2-$SCH_3$ | CH | CH(OH) | 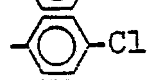 |
| 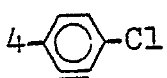 | N | CO | 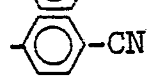 |
| 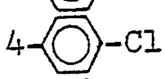 | CH | CO | 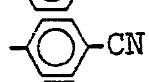 |
| 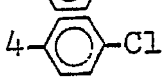 | N | CH(OH) | 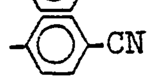 |
| 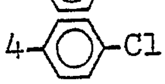 | CH | CH(OH) | 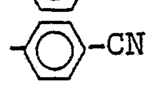 |
| 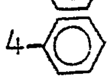 | N | CO | $-OCH_3$ |
| 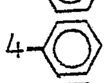 | CH | CO | $-OCH_3$ |
| 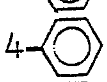 | N | CH(OH) | $-OCH_3$ |
| 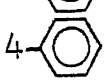 | CH | CH(OH) | $-OCH_3$ |
| 4-Cl | N | CO | 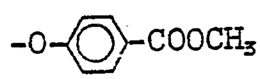 |
| 4-Cl | CH | CO | 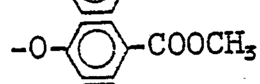 |
| 4-Cl | N | CH(OH) | 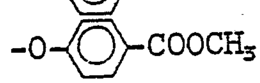 |
| 4-Cl | CH | CH(OH) | 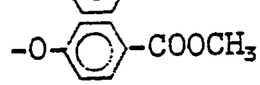 |

| $Z_n$ | A | B | R |
|---|---|---|---|
| 4-Cl | N | CO | 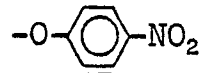 |
| 4-Cl | CH | CO | 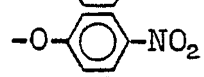 |
| 4-Cl | N | CH(OH) | 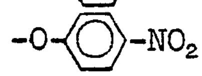 |
| 4-Cl | CH | CH(OH) | 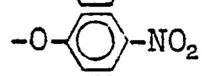 |
| 4-Cl,2-$CH_3$ | N | CO | 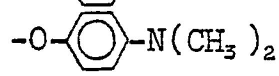 |
| 4-Cl,2-$CH_3$ | CH | CO | 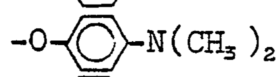 |
| 4-Cl,2-$CH_3$ | N | CH(OH) | 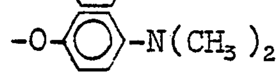 |
| 4-Cl,2-$CH_3$ | CH | CH(OH) | 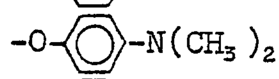 |
| 4-Cl | N | CO | 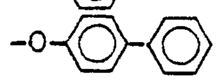 |
| 4-Cl | CH | CO | 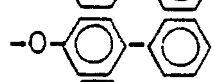 |
| 4-Cl | N | CH(OH) | 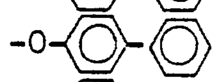 |
| 4-Cl | CH | CH(OH) | 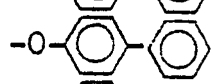 |
| 2,4-$Cl_2$ | N | CO | 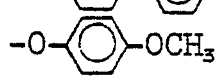 |
| 2,4-$Cl_2$ | CH | CO | 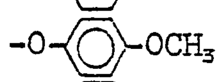 |
| 2,4-$Cl_2$ | N | CH(OH) | 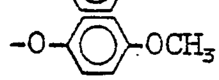 |
| 2,4-$Cl_2$ | CH | CH(OH) | 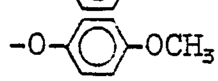 |
| 4-$NO_2$ | N | CO | 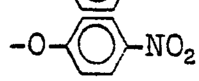 |
| 4-$NO_2$ | CH | CO | 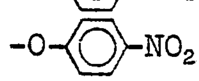 |
| 4-$NO_2$ | N | CH(OH) | 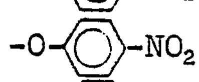 |
| 4-$NO_2$ | CH | CH(OH) | 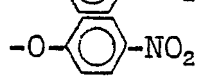 |
| 2,4-$Cl_2$ | N | CO | $-O-C_5H_{11}$ |
| 2,4-$Cl_2$ | CH | CO | $-O-C_5H_{11}$ |
| 2,4-$Cl_2$ | N | CH(OH) | $-O-C_5H_{11}$ |
| 2,4-$Cl_2$ | CH | CH(OH) | $-O-C_5H_{11}$ |

| $Z_n$ | A | B | R |
|---|---|---|---|
| $4-SO_2CH_3$ | N | CO | $-O-C_2H_5$ |
| $4-SO_2CH_3$ | CH | CO | $-O-C_2H_5$ |
| $4-SO_2CH_3$ | N | CH(OH) | $-O-C_2H_5$ |
| $4-SO_2CH_3$ | CH | CH(OH) | $-O-C_2H_5$ |
| $3-CF_3$ | N | CO | $-OCH_3$ |
| $3-CF_3$ | CH | CO | $-OCH_3$ |
| $3-CF_3$ | N | CH(OH) | $-OCH_3$ |
| $3-CF_3$ | CH | CH(OH) | $-OCH_3$ |
| $2,4,6-Cl_3$ | N | CO | $-OC_2H_5$ |
| $2,4,6-Cl_3$ | CH | CO | $-OC_2H_5$ |
| $2,4,6-Cl_3$ | N | CH(OH) | $-OC_2H_5$ |
| $2,4,6-Cl_3$ | CH | CH(OH) | $-OC_2H_5$ |
| 4-Cl | N | CO | $-SC_2H_5$ |
| 4-Cl | CH | CO | $-SC_2H_5$ |
| 4-Cl | N | CO | $-SO_2C_2H_5$ |
| 4-Cl | CH | CO | $-SO_2C_2H_5$ |
| 4-Cl | N | CO | $-S-CH(CH_3)_2$ |
| 4-Cl | CH | CO | $-S-CH(CH_3)_2$ |
| 4-Cl | N | CO | $-SO_2-CH(CH_3)_2$ |
| 4-Cl | CH | CO | $-SO_2-CH(CH_3)_2$ |
| 4-Cl | N | CO | $-SC_3H_7$ |
| 4-Cl | CH | CO | $-SC_3H_7$ |
| 4-Cl | N | CO | $-SO_2-C_3H_7$ |
| 4-Cl | CH | CO | $-SO_2-C_3H_7$ |

| $Z_n$ | A | B | R |
|---|---|---|---|
| 4-Cl | N | CO | $-S-C_4H_9$ |
| 4-Cl | CH | CO | $-S-C_4H_9$ |
| 4-Cl | N | CO | $-SO_2-C_4H_9$ |
| 4-Cl | CH | CO | $-SO_2-C_4H_9$ |
| 4-Cl | N | CO | -S 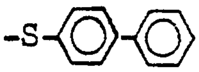 |
| 4-Cl | CH | CO | -S 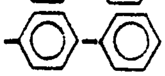 |
| 4-Cl | N | CO | $-SO_2$ 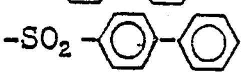 |
| 4-Cl | CH | CO | $-SO_2$ 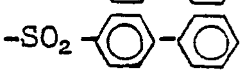 |
| 4-Cl | N | CH(OH) | $-SC_2H_5$ |
| 4-Cl | CH | CH(OH) | $-SC_2H_5$ |
| 4-Cl | N | CH(OH) | $-SO_2C_2H_5$ |
| 4-Cl | CH | CH(OH) | $-SO_2C_2H_5$ |
| 4-Cl | N | CH(OH) | $-S-CH(CH_3)_2$ |
| 4-Cl | CH | CH(OH) | $-S-CH(CH_3)_2$ |
| 4-Cl | N | CH(OH) | $-SO_2-CH(CH_3)_2$ |
| 4-Cl | CH | CH(OH) | $-SO_2-CH(CH_3)_2$ |
| 4-Cl | N | CH(OH) | $-SC_3H_7$ |
| 4-Cl | CH | CH(OH) | $-SC_3H_7$ |
| 4-Cl | N | CH(OH) | $-SO_2-C_3H_7$ |
| 4-Cl | CH | CH(OH) | $-SO_2-C_3H_7$ |
| 4-Cl | N | CH(OH) | $-S-C_4H_9$ |
| 4-Cl | CH | CH(OH) | $-S-C_4H_9$ |
| 4-Cl | N | CH(OH) | $-SO_2-C_4H_9$ |
| 4-Cl | CH | CH(OH) | $-SO_2-C_4H_9$ |

| $Z_n$ | A | B | R |
|---|---|---|---|
| 4-Cl | N | CH(OH) | 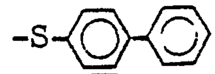 |
| 4-Cl | CH | CH(OH) | 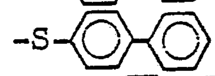 |
| 4-Cl | N | CH(OH) | 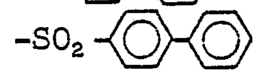 |
| 4-Cl | CH | CH(OH) | 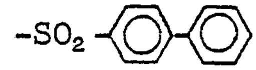 |
| 4-Cl | N | CO | 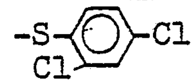 |
| 4-Cl | CH | CO | 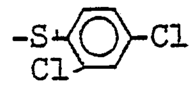 |
| 4-Cl | N | CO | 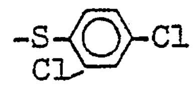 |
| 4-Cl | CH | CO | 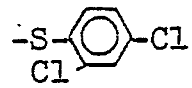 |
| 4-Cl | N | CO | 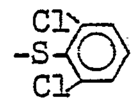 |
| 4-Cl | CH | CO | 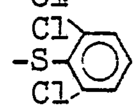 |
| 4-Cl | N | CO | 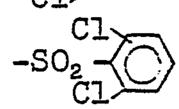 |
| 4-Cl | CH | CO | 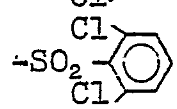 |
| 4-Cl | N | CH(OH) | 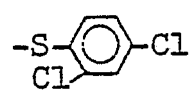 |
| 4-Cl | CH | CH(OH) | 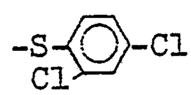 |
| 4-Cl | N | CH(OH) | 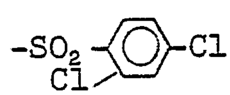 |
| 4-Cl | CH | CH(OH) | 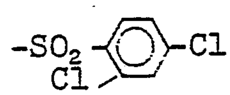 |

| $Z_n$ | A | B | R |
|---|---|---|---|
| 4-Cl | N | CH(OH) | 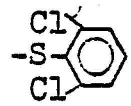 |
| 4-Cl | CH | CH(OH) | 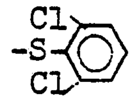 |
| 4-Cl | N | CH(OH) | 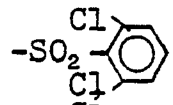 |
| 4-Cl | CH | CH(OH) | 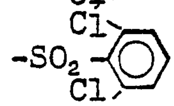 |
| $2,4-Cl_2$ | N | CO | 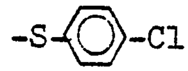 |
| $2,4-Cl_3$ | CH | CO | 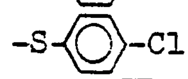 |
| $2,4-Cl_2$ | N | CO | 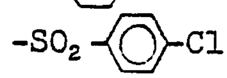 |
| $2,4-Cl_2$ | CH | CO | 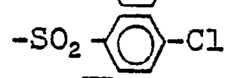 |
| $2,4-Cl_2$ | N | CO | 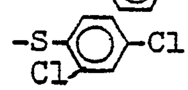 |
| $2,4-Cl_2$ | CH | CO | 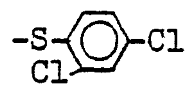 |
| $2,4-Cl_2$ | N | CO | 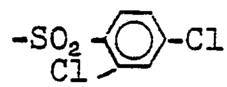 |
| $2,4-Cl_2$ | CH | CO | 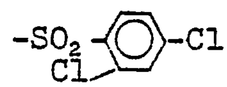 |
| $2,4-Cl_2$ | N | CO | 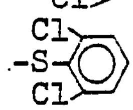 |
| $2,4-Cl_2$ | CH | CO | 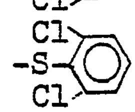 |
| $2,4-Cl_2$ | N | CO | 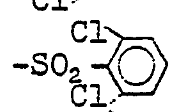 |
| $2,4-Cl_2$ | CH | CO | 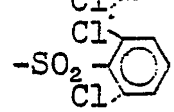 |
| $2,4-Cl_2$ | N | CH(OH) | 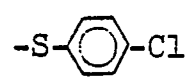 |
| $2,4-Cl_2$ | CH | CH(OH) | 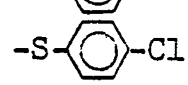 |

| $Z_n$ | A | B | R |
|---|---|---|---|
| 2,4-$Cl_2$ | N | CH(OH) | 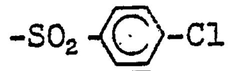 |
| 2,4-$Cl_2$ | CH | CH(OH) | 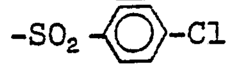 |
| 2,4-$Cl_2$ | N | CH(OH) | 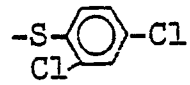 |
| 2,4-$Cl_2$ | CH | CH(OH) | 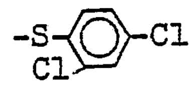 |
| 2,4-$Cl_2$ | N | CH(OH) | 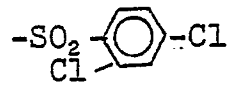 |
| 2,4-$Cl_2$ | CH | CH(OH) | 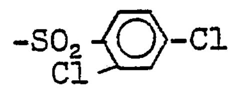 |
| 2,4-$Cl_2$ | N | CH(OH) | 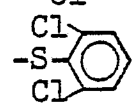 |
| 2,4-$Cl_2$ | CH | CH(OH) | 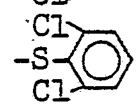 |
| 2,4-$Cl_2$ | N | CH(OH) | 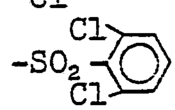 |
| 2,4-$Cl_2$ | CH | CH(OH) | 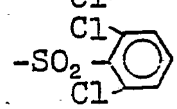 |
| 2,4-$Cl_2$ | N | CO | 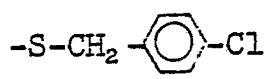 |
| 2,4-$Cl_2$ | CH | CO | 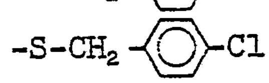 |
| 2,4-$Cl_2$ | N | CO | 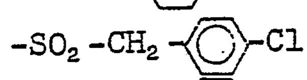 |
| 2,4-$Cl_2$ | CH | CO | 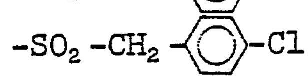 |
| 2,4-$Cl_2$ | N | CH(OH) | 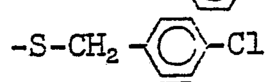 |
| 2,4-$Cl_2$ | CH | CH(OH) | 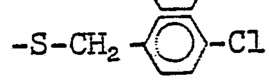 |
| 2,4-$Cl_2$ | N | CH(OH) | 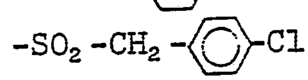 |
| 2,4-$Cl_2$ | CH | CH(OH) | 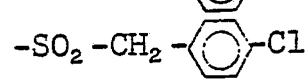 |
| 4-Cl | N | CO | $-SCH_3$ |
| 4-Cl | CH | CO | $-SCH_3$ |

| $Z_n$ | A | B | R |
|---|---|---|---|
| 4-Cl | N | CO | $-SO_2-CH_3$ |
| 4-Cl | CH | CO | $-SO_2-CH_3$ |
| 4-Cl | N | CH(OH) | $-S-CH_3$ |
| 4-Cl | CH | CH(OH) | $-S-CH_3$ |
| 4-Cl | N | CH(OH) | $-SO_2-CH_3$ |
| 4-Cl | CH | CH(OH) | $-SO_2-CH_3$ |
| 4-Cl | N | CO | $-S-C(CH_3)_3$ |
| 4-Cl | CH | CO | $-S-C(CH_3)_3$ |
| 4-Cl | N | CO | $-SO_2-C(CH_3)_3$ |
| 4-Cl | CH | CO | $-SO_2-C(CH_3)_3$ |
| 4-Cl | N | CH(OH) | $-S-C(CH_3)_3$ |
| 4-Cl | CH | CH(OH) | $-S-C(CH_3)_3$ |
| 4-Cl | N | CH(OH) | $-SO_2-C(CH_3)_3$ |
| 4-Cl | CH | CH(OH) | $-SO_2-C(CH_3)_3$ |

Verwendet man beispielsweise 1,4-Bis-(4-chlorphenoxy)-1-brom-3,3-dimethyl-butan-2-on und 1,2,4-Triazol als Ausgangsstoffe, so kann der Reaktionsablauf durch das folgende Formelschema wiedergegeben werden:

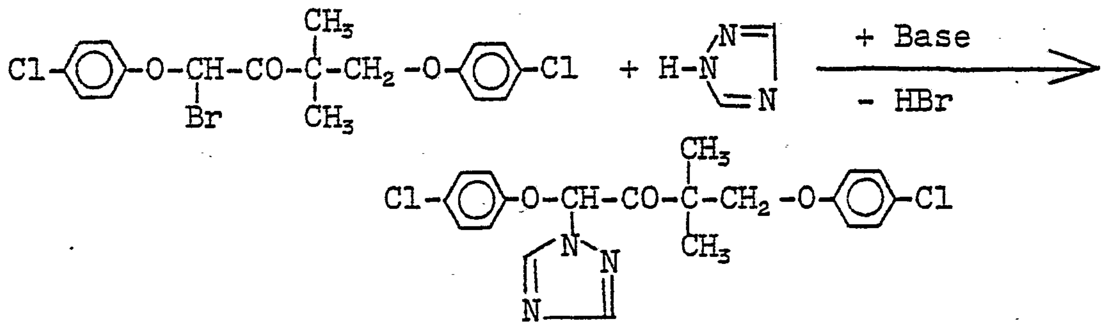


Verwendet man 1,4-Bis-(4-chlorphenoxy)-3,3-dimethyl-1-(1,2,4-triazol-1-yl)-butan-2-on und Natriumborhydrid als Ausgangsstoffe, so kann der Reaktionsablauf der Reduktion durch das folgende Formelschema wiedergegeben werden:

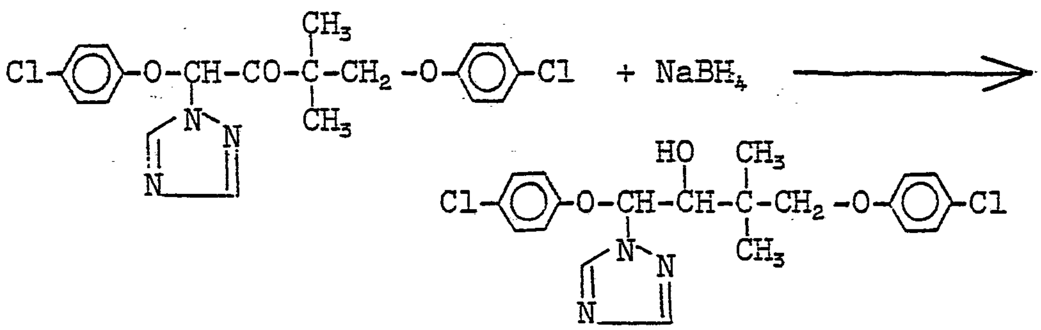


Verwendet man beispielsweise 1-(4-Chlorphenoxy)-4-(4-chlorphenylmercapto)-3,3-dimethyl-1-(1,2,4-triazol-1-yl)-butan-2-on und Wasserstoffperoxid in Eisessig als Ausgangsstoffe, so kann der Reaktionsablauf der Oxidation durch das folgende Formelschema wiedergegeben werden:

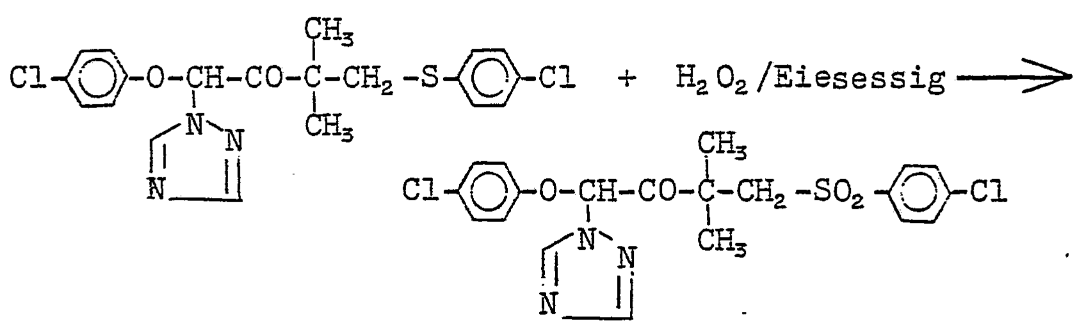

Die bei der Durchführung des erfindungsgemäßen Verfahrens als Ausgangsstoffe zu verwendenden Halogenetherketone sind durch die Formel (II) allgemein definiert. In dieser Formel steht $\underline{R}^2$ vorzugsweise für die Gruppierung $X^1-R^1$ sowie Cyano. $\underline{R}^1$ steht vorzugsweise für geradkettiges oder verzweigtes Alkyl mit 1 bis 6, insbesondere 1 bis 4 Kohlenstoffatomen sowie für gegebenenfalls substituiertes Aryl und Aralkyl mit jeweils 6 bis 10 Kohlenstoffatomen im Arylteil und 1 bis 2 Kohlenstoffatomen im Alkylteil, wie insbesondere Phenyl und Benzyl, wobei als Substituenten die bereits bei der Beschreibung der erfindungsgemäßen Stoffe der Formel (I) genannten Arylsubstituenten infrage kommen.

$\underline{X}^1$ steht vorzugsweise für Sauerstoff oder Schwefel.

<u>Z</u> und der Index <u>n</u> stehen vorzugsweise für diejenigen Reste, die bereits im Zusammenhang mit der Beschreibung der erfindungsgemäßen Stoffe der Formel (I) vorzugsweise für diesen Substituenten genannt wurden.

Die Halogenetherketone der Formel (II) sind noch nicht bekannt. Sie können jedoch nach bekannten Verfahren erhalten werden, indem man z.B. bekannte Phenole der Formel

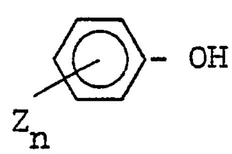 (IV)

in welcher

Z und n die oben angegebene Bedeutung haben,

mit einem Halogenetherketon der Formel

$$Hal'-CH_2-CO-\overset{\displaystyle CH_3}{\underset{\displaystyle CH_3}{\overset{|}{\underset{|}{C}}}}-CH_2-R^2 \qquad (V)$$

in welcher

$R^2$ die oben angegebene Bedeutung hat und

Hal' für Chlor oder Brom steht,

umsetzt. Das noch verbliebene aktive Wasserstoffatom wird anschließend in üblicher Weise gegen Halogen ausgetauscht (vergleiche auch die Herstellungsbeispiele). Die Halogenketone der Formel (II) können gegebenenfalls ohne Isolierung direkt weiter umgesetzt werden.

Die Halogenketone der Formel (V) sind ebenfalls noch nicht bekannt. Sie können jedoch nach bekannten Verfahren erhalten werden, indem man 4-substituierte 3,3-Dimethyl-butan-2-one der Formel

$$CH_3 - CO - \underset{CH_3}{\overset{CH_3}{\underset{|}{\overset{|}{C}}}} - CH_2 - R^2 \qquad (VI)$$

in welcher

$R^2$ die oben angegebene Bedeutung hat,

mit Chlor oder Brom in Gegenwart eines inerten organischen Lösungsmittels, wie beispielsweise Ether, chlorierten oder nicht chlorierten Kohlenwasserstoffen, bei Raumtemperatur umsetzt (vergleiche auch die Herstellungsbeispiele), oder mit üblichen Chlorierungsmitteln, wie beispielsweise Sulfurylchlorid, bei 20 bis 60°C umsetzt.

Die 4-substituierten 3,3-Dimethyl-butan-2-one der Formel (VI) sind teilweise bekannt (vergleiche J.Org.Chem. 42, 1709-1717 (1977); J.Am.Chem.Soc. 98, 7882-84 (1976); J. Org.Chem. 37, 2834-2840 (1972) sowie C.A. 82, 30 898 j (1975)), bzw. können sie gemäß einem eigenen Vorschlag hergestellt werden, indem man z.B. Butan-2-on-Derivate der Formel

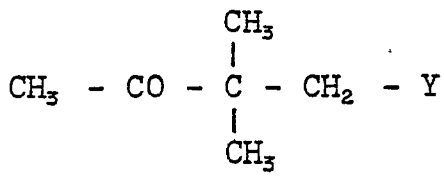 (VII)

in welcher

Y für Chlor, Brom oder die Gruppierung $-O-SO_2-R^3$ steht, wobei

$R^3$ für Alkyl mit 1 bis 4 Kohlenstoffatomen sowie für gegebenenfalls durch Alkyl mit 1 bis 4 Kohlenstoffatomen substituiertes Phenyl steht,

mit Verbindungen der Formel

$$Me - R^2 \qquad (VIII)$$

in welcher

$R^2$ die oben angegebene Bedeutung hat und

Me für ein Alkalimetall, wie vorzugsweise Natrium und Kalium, oder Wasserstoff steht,

in Gegenwart eines organischen Lösungsmittels, wie beispielsweise Glykol oder Dimethylformamid und gegebenenfalls in Gegenwart eines Säurebindemittels, wie beispielsweise Natriumcarbonat, bei Temperaturen zwischen 80 und 150°C umsetzt (vergleiche auch die Herstellungsbeispiele), oder indem man das 3,3-Dimethyl-4-hydroxy-butan-2-on der Formel

$$\begin{array}{c} \quad\quad\quad\quad\quad\quad CH_3 \\ \quad\quad\quad\quad\quad\quad | \\ CH_3 - CO - C - CH_2 - O - H \\ \quad\quad\quad\quad\quad\quad | \\ \quad\quad\quad\quad\quad\quad CH_3 \end{array} \qquad (IX)$$

mit Verbindungen der Formel

$$Y' - R^4 \qquad (X)$$

in welcher

$R^4$ für Alkyl mit 1 bis 6 , vorzugsweise 1 bis 4 Kohlenstoffatomen oder für gegebenenfalls substituiertes Benzyl steht, wobei als Substituenten die weiter oben genannten Substituenten infrage kommen und

Y' für Chlor, Brom oder die Gruppierung $-O-SO_2-R^5$ steht, wobei

$R^5$ für Alkyl und Alkoxy mit 1 bis 4 Kohlenstoffatomen sowie für gegebenenfalls durch Alkyl mit 1 bis 4 Kohlenstoffatomen substituiertes Phenyl steht,

gegebenenfalls in Gegenwart von Wasser oder eines organischen Lösungsmittels und gegebenenfalls in Gegenwart eines Säurebindemittels bei Temperaturen zwischen 20 und 100°C umsetzt.

Die Butan-2-on-Derivate der Formel (VII) sind bekannt (vergleiche z.B. DE-OS 26 32 603 [LeA 17 273] und Journal Org.Chem. 35. 2391 (1970)) , bzw, können sie in allgemein bekannter Art und Weise aus dem 3,3-Dimethyl-4-hydroxy-butan-2-on erhalten werden (vergleiche auch die Herstellungsbeispiele).

Das 3,3 -Dimethyl-4-hydroxy-butan-2-on der Formel (IX) ist ebenfalls bekannt (vergleiche z.B. DE-OS 26 32 603).

Die Verbindungen der Formel (VIII) und (X) sind allgemein bekannte Verbindungen der organischen Chemie. Die Verbindungen der Formel (VIII) werden gegebenenfalls in-situ eingesetzt.

Die außerdem für das erfindungsgemäße Verfahren als Ausgangsstoffe zu verwendenden Azole sind durch die Formel (III) allgemein definiert. In dieser Formel steht A vorzugsweise für Stickstoff oder die CH-Gruppe.

Die Azole der Formel (III) sind allgemein bekannte Verbindungen der organischen Chemie.

Die für die gegebenenfalls durchzuführende Reduktion als Ausgangsstoffe zu verwendenden Keto-Derivate sind durch die Formel (Ia) allgemein definiert. In dieser Formel stehen A, $R^2$, Z und der Index n vorzugsweise für diejenigen Reste, die bereits im Zusammenhang mit der Beschreibung der erfindungsgemäßen Stoffe der Formel (I) vorzugsweise für diese Substituenten genannt wurden.

Die für die gegebenenfalls durchzuführende Oxidation als Ausgangsstoffe zu verwendenden Thio-Derivate sind durch die Formel (Ib) allgemein definiert. In dieser Formel steht $R^1$ vorzugsweise für geradkettiges oder verzweigtes Alkyl mit 1 bis 6 Kohlenstoffatomen sowie für gegebenenfalls substituiertes Phenyl und Benzyl, wobei als Substituenten vorzugsweise die bereits weiter oben genannten Substituenten infrage kommen. A, B, Z und der Index n stehen vorzugsweise für diejenigen Reste, die bereits im Zusammenhang mit der Beschreibung der erfindungsgemäßen Stoffe der Formel (I) vorzugsweise für diese Substituenten genannt wurden.

Die Keto-Derivate der Formel (Ia) und die Thio-Derivate der Formel (Ib) sind erfindungsgemäße Verbindungen.

Für die erfindungsgemäße Umsetzung kommen als Verdünnungsmittel inerte organische Lösungsmittel infrage. Hierzu gehören vorzugsweise Ketone, wie Diethylketon und insbesondere Aceton und Methylethylketon; Nitrile, wie Propionitril, insbesondere Acetonitril; Alkohole, wie Ethanol oder Isopropanol; Ether, wie Tetrahydrofuran oder Dioxan; Benzol; Toluol; Formamide, wie insbesondere Dimethylformamid; und halogenierte Kohlenwasserstoffe.

Die erfindungsgemäße Umsetzung wird in Gegenwart eines Säurebindemittels vorgenommen. Man kann alle üblicherweise verwendbaren anorganischen oder organischen Säurebinder zugeben, wie Alkalicarbonate, beispielsweise Natriumcarbonat, Kaliumcarbonat und Natriumhydrogencarbonat, oder wie niedere tertiäre Alkylamine, Cycloalkylamine oder Aralkylamine, beispielsweise Triethylamin, N,N-Dimethylcyclohexylamin, Dicyclohexylamin, N,N-Dimethylbenzylamin, weiterhin Pyridin und Diazabicycloctan.

Vorzugsweise verwendet man einen entsprechenden Ueberschuß an Azol.

Die Reaktionstemperaturen können in einem größeren Bereich variiert werden. Im allgemeinen arbeitet man zwischen etwa 20 bis etwa 150°C, vorzugsweise bei 60 bis 120°C. Bei Anwesenheit eines Lösungsmittels wird zweckmäßigerweise beim Siedepunkt des jeweiligen Lösungsmittels gearbeitet.
Bei der Durchführung des erfindungsgemäßen Verfahrens setzt man auf 1 Mol der Verbindungen der Formel (II) vorzugsweise 2 bis 4 Mol Azol und 1 bis 4 Mol Säurebinder ein. Zur Isolierung der Verbindungen der Formel (I) wird das Lösungsmittel abdestilliert, der Rückstand mit

einem organischen Solvens aufgenommen und mit Wasser gewaschen. Die organische Phase wird über Natriumsulfat getrocknet und im Vakuum vom Lösungsmittel befreit. Der Rückstand wird durch Destillation bzw. Umkristallisation oder Salzbildung und Umkristallisation gereinigt.

Die erfindungsgemäße Reduktion erfolgt in üblicher Weise, wie z.B. durch Umsetzung mit komplexen Hydriden, gegebenenfalls in Gegenwart eines Verdünnungsmittels, oder durch Umsetzung mit Aluminium-isopropylat in Gegenwart eines Verdünnungsmittels.

Arbeitet man mit komplexen Hydriden, so kommen als Verdünnungsmittel für die erfindungsgemäße Umsetzung polare organische Lösungsmittel infrage. Hierzu gehören vorzugsweise Alkohole, wie Methanol, Ethanol, Butanol, Isopropanol, und Ether, wie Diethylether oder Tetrahydrofuran. Die Reaktion wird im allgemeinen bei 0 bis 30°C, vorzugsweise bei 0 bis 20°C durchgeführt. Hierzu setzt man auf 1 Mol des Ketons der Formel (Ia) etwa 1 Mol eines komplexen Hydrids, wie Natriumborhydrid oder Lithiumalanat, ein. Zur Isolierung der reduzierten Verbindungen der Formel (I) wird der Rückstand in verdünnter Salzsäure aufgenommen, anschließend alkalisch gestellt und mit einem organischen Lösungsmittel extrahiert. Die weitere Aufarbeitung erfolgt in üblicher Weise.

Arbeitet man mit Aluminiumisopropylat, so kommen als Verdünnungsmittel für die erfindungsgemäße Umsetzung bevorzugt Alkohole, wie Isopropanol, oder inerte Kohlenwasserstoffe, wie Benzol, infrage. Die Reaktionstemperaturen können wiederum in einem größeren Bereich variiert werden; im allgemeinen arbeitet man zwischen 20 und 120°C, vorzugsweise bei 50 bis 100°C. Zur Durchführung der Reaktion setzt man auf 1 Mol des Ketones der Formel (Ia) etwa 0,3 bis 2 Mol Aluminiumisopropylat ein. Zur Isolierung der reduzierten Verbindungen der Formel (I) wird das überschüssige Lösungsmittel im

Vakuum entfernt und die entstandenen Aluminium-Verbindungen mit verdünnter Schwefelsäure oder Natronlauge zersetzt. Die weitere Aufarbeitung erfolgt in üblicher Weise.

Die erfindungsgemäße Oxidation erfolgt durch die Umsetzung mit üblichen anorganischen oder organischen Oxidationsmitteln. Hierzu gehören vorzugsweise organische Persäuren, wie z.B. Peressigsäure, p-Nitroperbenzoesäure, m-Chlorperbenzoesäure; anorganische Persäuren, wie z.B. Perjodsäure; weiterhin Wasserstoffperoxid in Eisessig oder Methanol, Kaliumpermanganat und Chromsäure.

Die Reaktionstemperaturen können bei der Durchführung der Oxidation in einem größeren Bereich variiert werden. Im allgemeinen arbeitet man zwischen etwa -50 bis 100 °C, vorzugsweise zwischen -30 und 80°C.

Bei der Durchführung der erfindungsgemäßen Oxidation setzt man auf 1 Mol der erfindungsgemäßen Verbindungen der Formel (Ib) etwa 1 bis 5 Mol Oxidationsmittel ein. Bei der Anwendung von 1 Mol Oxidationsmittel, wie m-Chlorperbenzoesäure in Methylenchlorid oder Wasserstoffperoxid in Acetanhydrid bei Temperaturen zwischen -30 bis +30°C, entstehen vorzugsweise die erfindungsgemäßen Verbindungen der Formel (I) mit X=SO. Bei Ueberschuß an Oxidationsmittel und höheren Temperaturen (10 bis 80°C) entstehen vorzugsweise die erfindungsgemäßen Verbindungen der Formel (I) mit X = $SO_2$. Die Isolierung der Oxidationsprodukte erfolgt in üblicher Weise.

Zur Herstellung von physiologisch verträglichen Säureadditionssalzen der Verbindungen der Formel (I) kommen vorzugsweise folgende Säuren infrage: Die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure, sowie Sulfonsäuren,wie z.B. p-Toluolsulfonsäure und 1,5-Naphthalindisulfonsäure.
Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I. und II. sowie IV. bis VIII. Nebengruppe infrage, wobei Kupfer, Zink, Mangan, Magnesium, Zinn, Eisen und Nickel beispielhaft genannt seien.
Als Anionen der Salze kommen solche in Betracht, die sich vorzugsweise von folgenden Säuren ableiten: Halogenwasserstoffsäuren, wie z.B. die Chlorwasserstoffsäure und die Bromwasserstoffsäure, ferner Phosphorsäure, Salpetersäure und Schwefelsäure.

Die Metallsalz-Komplexe von Verbindungen der Formel (I) können in einfacher Weise nach üblichen Verfahren erhalten werden, so z.B.durch Lösen des Metallsalzes in Alkohol,z.B.Ethanol und Hinzufügen zur Verbindung der Formel (I).Man kann Metallsalz-Komplexe in bekannter Weise,z.B. durch Abfiltrieren,Isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Getreidekrankheiten, wie Mehltau und Rost, von Venturia-Arten, wie gegen den Erreger des Apfelschorfs (Fusicladium dendriticum), von Uromyces-Arten, wie gegen den Erreger des Bohnenrostes (Uromyces phaseoli), sowie von Erysiphe-Arten, wie gegen den Erreger des echten Gurkenmehltaus (Erysiphe cichoracearum) eingesetzt werden. Die erfindungsgemäßen Wirkstoffe zeigen auch eine allgemein gute in-vitro-Wirkung.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.



BAD ORIGINAL

Herstellungsbeispiele

Beispiel 1

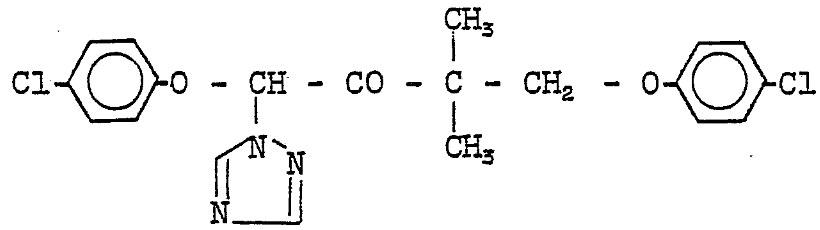

21,6g (0,05 Mol) rohes 1,4-Bis-(4-chlorphenoxy)-1-brom-3,3-dimethyl-butan-2-on werden mit 14 g (0,2 Mol) 1,2,4-Triazol in 100 ml Acetonitril 17 Stunden unter Rückfluß gerührt. Danach wird durch Abdestillieren des Lösungsmittels unter vermindertem Druck eingeengt. Der Rückstand wird in 400 ml Methylenchlorid aufgenommen und dreimal mit je 800 ml Wasser ausgeschüttelt, über Natriumsulfat getrocknet und eingeengt. Der Rückstand wird in 100 ml Aceton aufgenommen und tropfenweise mit 9g (0,05 Mol) 1,5-Naphthalindisulfonsäure-tetrahydrat in 50 ml Aceton versetzt. Der entstehende Niederschlag wird abgesaugt und in 200 ml Methylenchlorid suspendiert. Man gibt 400 ml gesättigte Natriumhydrogencarbonatlösung zu, verrührt 30 Minuten, trennt die organische Phase ab, trocknet über Natriumsulfat und engt durch Abdestillieren des Lösungsmittels ein. Man erhält 12,3g (58,6 % der Theorie) 1,4-Bis-(4-chlorphenoxy)-3,3-dimethyl-1-(1,2,4-triazol-1-yl)-butan-2-on als Oel.

Das entsprechende 1,5-Naphthalindisulfonat-Salz hat einen Schmelzpunkt von 206-08°C.

Herstellung der Vorstufen

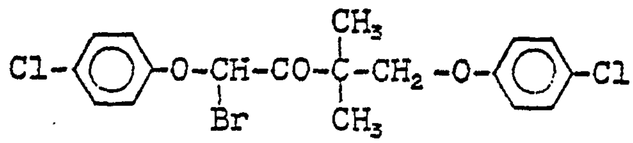

36,6g (o,1 Mol) 1,4-Bis-(4-chlorphenoxy)-3,3-dimethylbutan-2-on werden in 250 ml Chloroform gelöst und bei 20°C tropfenweise so mit 16,6g (0,1 Mol) Brom versetzt, daß laufend Entfärbung eintritt . Nach beendeter Zugabe läßt man 30 Minuten bei Raumtemperatur nachrühren und engt danach durch Abdestillieren des Lösungsmittels im Vakuum ein. Man erhält 43,2g (quantitativer Umsatz) 1,4-Bis-(4-chlorphenoxy)-1-brom-3,3-dimethyl-butan-2-on als Oel, das direkt weiter umgesetzt wird.

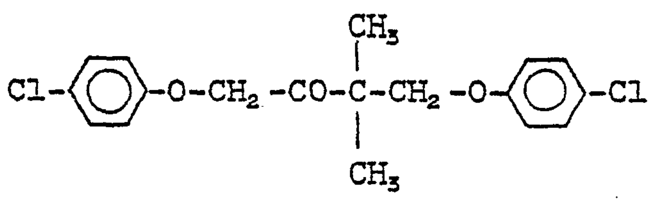

20,6g (0,16 Mol) 4-Chlorphenol und 28g (0,2 Mol) Kaliumcarbonat werden in 250 ml Aceton gelöst. Unter Rückfluß werden langsam 48,5g 1-Brom-4-(4-chlorphenoxy)-3,3-dimethyl-butan-2-on in 50 ml Aceton zugetropft. Nach beendeter Zugabe läßt man 15 Stunden unter Rückfluß rühren, filtriert und engt das Filtrat ein. Der Rückstand wird in 500ml Methylenchlorid aufgenommen, mit 200 ml Wasser , 200 ml gesättigter Natriumhydrogencarbonatlösung und nochmals mit 200 ml Wasser ausgeschüttelt.

Die organische Phase wird über Natriumsulfat getrocknet, eingeengt und der Rückstand in 150 ml Diisopropylether aufgenommen. Nach dem Einengen erhält man 36,6g (65 % der Theorie) 1,4-Bis-(4-chlorphenoxy)-3,3-dimethyl-butan-2-on als farblose Kristalle vom Schmelzpunkt 76-77°c.

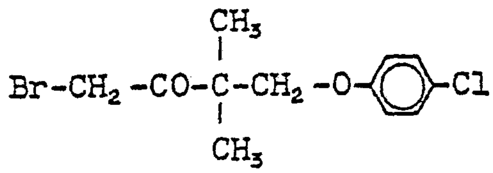

36g (0,159 Mol) 1-(4-Chlorphenoxy)-2,2-dimethyl-butan-3-on werden in 300 ml Chloroform gelöst und bei 20°C tropfenweise so mit 25,5g (0,159 Mol) Brom versetzt, daß laufend Entfärbung eintritt. Nach beendeter Zugabe läßt man 30 Minuten bei Raumtemperatur rühren und engt danach durch Abdestillieren des Lösungsmittels im Vakuum ein. Man erhält 48,5g (quantitativer Umsatz) rohes 1-Brom-4-(4-chlorphenoxy)-3,3-dimethyl-butan-2-on als Oel.

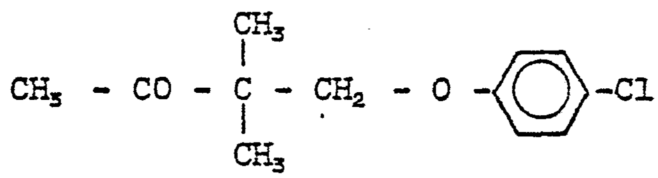

29,7g (0,55 Mol) Natriummethylat werden in 500 ml Methanol gelöst und unter Rühren mit 70,4g (0,55 Mol) 4-Chlorphenol versetzt. Nach 10 Minuten Rühren wird das Lösungsmittel unter vermindertem Druck abdestilliert und der Rückstand in 100 ml Glykol aufgenommen. Diese Lösung wird zu einer Lösung von 135g (0,5 Mol) 2,2-Dimethyl-1-tosyloxy-butan-3-on in 200 ml Glykol gegeben. Man läßt 48 Stunden bei 100 bis 120°C rühren, kühlt ab und rührt das Reaktionsgemisch in 2000 ml Wasser ein. Man extrahiert zweimal mit je 250 ml Diethylether, wäscht die vereinigten organischen Phasen dreimal mit je 100 ml Wasser, einmal mit 100 ml 10%-iger Natronlauge und nochmals mit 100 ml Wasser, trocknet über Natriumsulfat und destilliert. Man erhält 62,9g (55,7% der Theorie) 1-(4-Chlorphenoxy)-2,2-dimethyl-butan-3-on vom Siedepunkt 135-140°C/0,4 Torr.



BAD ORIGINAL

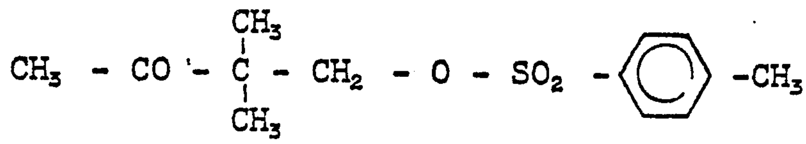

47,6g (0,25 Mol) 4-Toluolsulfochlorid werden in 100 ml Chloroform gelöst, 35g (0,3 Mol) 2,2-Dimethyl-1-hydroxy-butan-3-on zugegeben und bei 0 bis 5°C 40 ml (0,5 Mol) Pyridin zugetropft. Man läßt 15 Stunden bei Raumtemperatur nachrühren, gibt das Reaktionsgemisch auf 200 g Eis und 70 ml konzentrierter Salzsäure, trennt die organische Phase ab, wäscht sie dreimal mit je 200 ml Wasser nach, trocknet über Natriumsulfat und engt ein. Der Rückstand wird in 100 ml Petrolether aufgenommen, wobei das Endprodukt auskristallisiert. Man erhält 48g (71 % der Theorie) 2,2-Dimethyl-1-tosyloxy-butan-3-on als farblose Kristalle vom Schmelzpunkt 49-52°C.

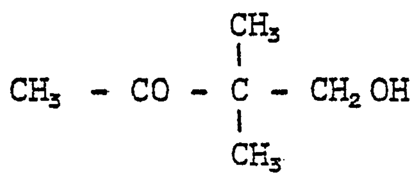

Zu 172g (2 Mol) Methylisopropylketon in 1000 ml Methanol werden 66g (2,2 Mol) Paraformaldehyd und 1 g Kaliumhydroxid in 10 ml Methanol getropft. Man erhitzt 15 Stunden unter Rückfluß und destilliert anschließend das Methanol über eine Kolonne bei 82°C Innentemperatur ab. Der Rückstand wird im Wasserstrahlvakuum destilliert. Man erhält 152,7g (68 % der Theorie) 2,2-Dimethyl-1-hydroxy-butan-3-on vom Siedepunkt 80-82°C/12 Torr.



BAD ORIGINAL

Beispiel 2



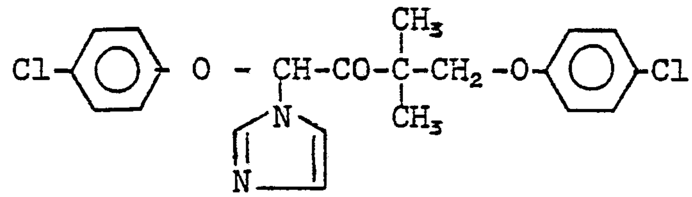

21,6g (0,05 Mol) rohes 1,4-Bis-(4-chlorphenoxy)-1-brom-3,3-dimethyl-butan-2-on werden mit 14 g (0,2 Mol) Imidazol in 100 ml Acetonitril 17 Stunden unter Rückfluß gerührt. Danach wird durch Abdestillieren des Lösungsmittels unter vermindertem Druck eingeengt. Der Rückstand wird in 400 ml Methylenchlorid aufgenommen, dreimal mit je 800 ml Wasser ausgeschüttelt, über Natriumsulfat getrocknet und eingeengt. Der Rückstand wird in 100 ml Aceton aufgenommen und tropfenweise mit 9g (0,05 Mol) 1,5-Naphthalindisulfonsäure-tetrahydrat in 50 ml Aceton versetzt. Der entstehende Niederschlag wird abgesaugt und in 200 ml Methylenchlorid suspendiert. Man gibt 400 ml gesättigte Natriumhydrogencarbonatlösung zu, verrührt 30 Minuten und trennt die organische Phase ab. Nach Abdestillieren des Lösungsmittels erhält man 14,1g (67 % der Theorie) 1,4-Bis-(4-chlorphenoxy)-3,3-dimethyl-1-(imidazol-1-yl)-butan-2-on als Oel.

Das entsprechende 1,5-Naphthalindisulfonat-Salz hat den Schmelzpunkt 214-17°C.

Beispiel 3

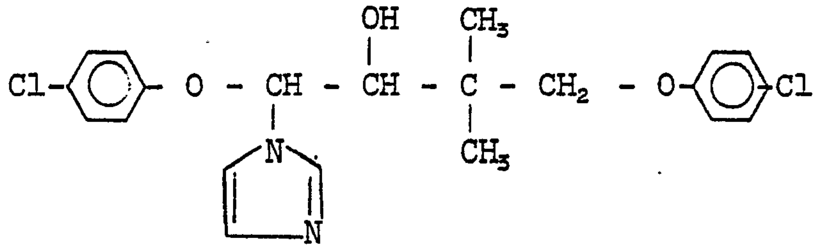

13,1g(0,031 Mol) 1,4-Bis-(4-chlorphenoxy)-3,3-dimethyl-1-(imidazol-1-yl)-butan-2-on(Beispiel 2) werden in 100 ml Methanol gelöst und bei 0 bis 5°C portionsweise mit 1,4g Natriumborhydrid versetzt. Man läßt 5 Stunden bei Raumtemperatur nachrühren, säuert mit 10 ml 2n Salzsäure an und läßt weitere 2 Stunden bei Raumtemperatur rühren. Danach wird das Lösungsmittel im Vakuum abdestilliert und der Rückstand in 300 ml Methylenchlorid aufgenommen. Man verrührt mit 200 ml gesättigter Natriumhydrogencarbonatlösung, trennt die organische Phase ab, wäscht sie zweimal mit je 300 ml Wasser, trocknet über Natriumsulfat und destilliert das Lösungsmittel im Vakuum ab. Der farblose Rückstand wird in 50 ml Diisopropylether aufgenommen und die entstehenden Kristalle abgesaugt. Man erhält 10,8g (82,7 % der Theorie) 1,4-Bis-(4-chlorphenoxy)-3,3-dimethyl-1-(imidazol-1-yl)-butan-2-ol als Diastereomerengemisch vom Schmelzpunkt 142-44°C.

Beispiel 4

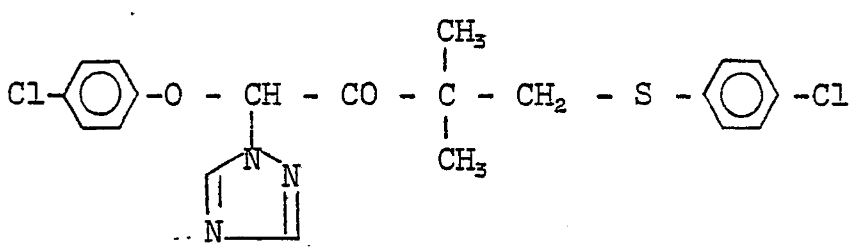

Zu 110,5g (0,3 Mol) 1-(4-Chlorphenoxy)-4-(4-chlorphenyl mercapto)-3,3-dimethyl-butan-2-on in 750 ml Tetrachlorkohlenstoff tropft man bei Raumtemperatur 48g (0,3 Mol) Brom. Man läßt eine Stunde bei Raumtemperatur nachrühren, engt durch Abdestillieren des Lösungsmittels im Vakuum ein und nimmt den Rückstand in 750 ml Toluol auf. Zu dieser Lösung werden 73g (1,06 Mol) 1,2,4-Triazol und 150g (1,06 Mol) Kaliumcarbonat gegeben. Man erhitzt 8 Stunden auf 90°C, läßt auf Raumtemperatur abkühlen und trennt die organische Phase ab. Diese wird mit verdünnter Natronlauge extrahiert, mit Wasser nachgewaschen, über Natriumsulfat getrocknet und im Vakuum eingeengt. Man erhält 34g (26 % der Theorie) 1-(4-Chlorphenoxy)-4-(4-chlorphenylmercapto)-3,3-dimethyl-1-(1,2,4-triazol-1-yl)-butan-2-on vom Schmelzpunkt 115°C.

Herstellung der Vorstufen

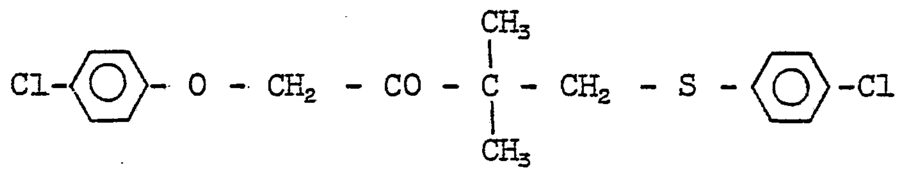

133,7g (0,41 Mol) 1-Brom-4-(4-chlorphenylmercapto)-3,3-dimethyl-butan-2-on werden mit 64g (0,5 Mol) 4-Chlorphenol und 69g (0,5 Mol) Kaliumcarbonat in 500 ml Toluol 5 Stunden auf 100°C erhitzt. Danach läßt man abkühlen und filtriert. Das Filtrat wird mit verdünnter Natronlauge ausgeschüttelt, mit Wasser nachgewaschen, über Natriumsulfat getrocknet und durch Abdestillieren des Lösungsmittels im Vakuum eingeengt. Man erhält 110,5g (72 % der Theorie) 1-(4-Chlorphenoxy)-4-(4-chlorphenylmercapto)-3,3-dimethyl-butan-2-on als gelbliches Oel vom Brechungsindex $n_D^{20}$ = 1,5932.

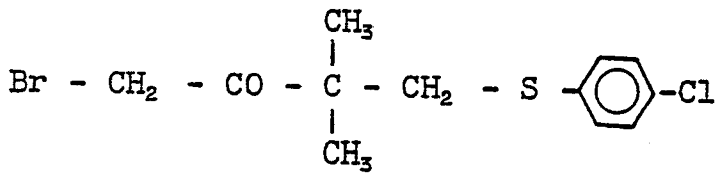

97g (0,4 Mol) 1-(4-Chlorphenylmercapto)-2,2-dimethylbutan-3-on werden in 400 ml Tetrachlorkohlenstoff gelöst und innerhalb von 30 Minuten tropfenweise mit 64 g (0,4 Mol) Brom versetzt. Man läßt 1 Stunde bei Raumtemperatur nachgerühren und engt dann durch Abdestillieren des Lösungsmittels ein. Man erhält 127g ( 99% der Theorie) rohes 1-Brom-4-(4-chlorphenylmercapto)-3,3-dimethylbutan-2-on als gelbliches Oel, das direkt weiter umgesetzt wird.

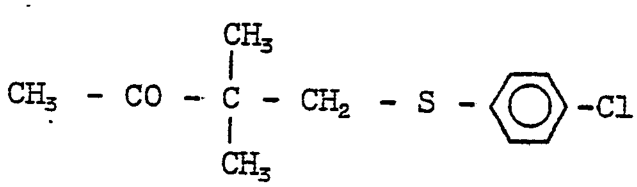

134,5g (1 Mol) 4-Chlorpinakolin werden mit 216,7g (1,5 Mol) 4-Chlorthiophenol und 210g (1,52 Mol) Kaliumcarbonat in 500 ml Dimethylformamid 15 Stunden bei 150°C gerührt. Man läßt auf Raumtemperatur abkühlen, verrührt mit 10 l Wasser und extrahiert mit Ether. Die Etherphase wird über Natriumsulfat getrocknet, eingeengt und der

Rückstand im Vakuum destilliert. Man erhält 189g (78 % der Theorie) 1-(4-Chlormethylmercapto)-2,2-dimethyl-butan-3-on vom Siedepunkt 146°C/0.5 Torr.

$$\begin{array}{c} \qquad\qquad\qquad CH_3 \\ \qquad\qquad\qquad | \\ CH_3 - CO - C - CH_2Cl \\ \qquad\qquad\qquad | \\ \qquad\qquad\qquad CH_3 \end{array}$$

11,6g (0,1 Mol) 2,2-Dimethyl-1-hydroxy-butan-3-on (Herstellung vgl.Bsp.1) werden bei 50 bis 60°C (Eiskühlung) zu 20,5g (0,1 Mol) N,N-Diethyl-1,2,2-trichlorvinyl-amin getropft. Nach zweistündigem Rühren bei 60°C wird im Wasserstrahlvakuum destilliert. Man erhält 8,1g (60 % der Theorie) 1-Chlor-2,2-dimethyl-butan-3-on vom Schmelzpunkt 60-62°C/12 Torr.
(Das 1-Chlor-2,2-dimethyl-butan-3-on wird in einer Ausbeute von 70 % erhalten, wenn man äquimolare Mengen 2,2-Dimethyl-1-hydroxy-butan-3-on und Triphenylphosphin in der zehnfachen Menge Tetrachlorkohlenstoff 12 Stunden unter Rückfluß erhitzt, das Lösungsmittel abdestilliert, den Rückstand in Ether aufnimmt, filtriert und destilliert.)

Beispiel 5

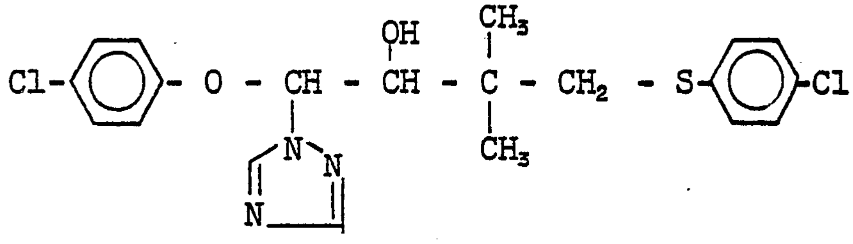

10g (0,023 Mol) 1-(4-Chlorphenoxy)-4-(4-chlorphenylmercapto)-3,3-dimethyl-1-(1,2,4-triazol-1-yl)-butan-2-on (Beispiel 4) in 200 ml Methanol werden portionsweise mit 0,35g (0,0092 Mol) Natriumborhydrid in 8 ml Wasser versetzt. Man läßt 4 Stunden bei 20°C nachrühren, gießt das Reaktionsgemisch auf Wasser und extrahiert mit Ether. Die Etherphase wird über Natriumsulfat getrocknet und eingeengt. Man erhält 5,5g (54,6 % der Theorie) 1-(4-Chlorphenoxy)-4-(4-chlorphenylmercapto)-3,3-dimethyl-1-(1,2,4-triazol-1-yl)-butan-2-ol vom Schmelzpunkt 132°C.

Beispiel 6

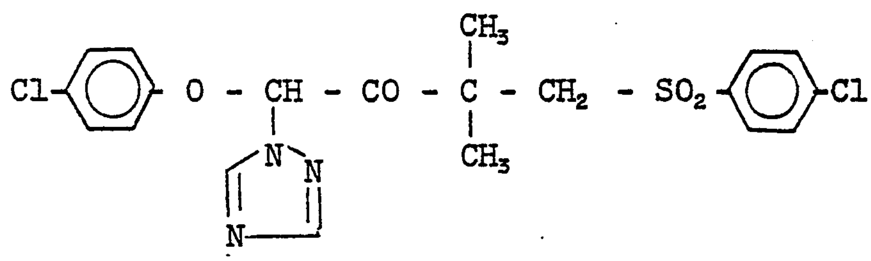

10g (0,023 Mol) 1-(4-Chlorphenoxy)-4-(4-chlorphenylmercapto)3,3-dimethyl-1-(1,2,4-triazol-1-yl)-butan-2-on (Beispiel 4) in 70 ml Eisessig werden mit 14,2g (0,125 Mol) 30%-igem Wasserstoffperoxid 5 Stunden bei 50°C gerührt. Danach wird das Reaktionsgemisch in 250 ml Wasser gegeben und mit Ether extrahiert. Die Etherphase wird mit Natriumhydrogencarbonatlösung ausgeschüttelt, mit Wasser nachgewaschen, über Natriumsulfat getrocknet und eingeengt. Man erhält 8,4g(74 % der Theorie) 1-(4-Chlorphenoxy)-4-(4-chlorphenylsulfonyl)-3,3-dimethyl-1-(1,2,4-triazol-1-yl)-butan-2-on vom Schmelzpunkt 55-60°C.

In entsprechender Weise werden die nachfolgenden Beispiele der allgemeinen Formel

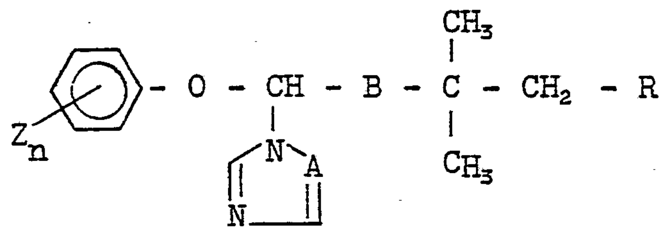 (I)

erhalten:

| Bsp.Nr. | $Z_n$ | A | B | R | Schmelzpunkt(°C) |
|---|---|---|---|---|---|
| 7 | 2,4-$Cl_2$ | N | CO | 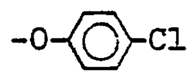 | 212(xHCl) |
| 8 | 2,4-$Cl_2$ | N | CO | 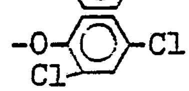 | 140-42 (xHCl) |
| 9 | 4-Cl | N | CO | 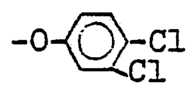 | 40 |
| 10 | 4-Cl | N | CO | $-O-C_2H_5$ | 67-68 |
| 11 | 4-Cl | N | CO | $-O-CH_3$ | Oel |
| 12 | 4-Cl | N | CH(OH) | 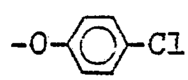 | 105 |
| 13 | 4-Cl | N | CH(OH) | 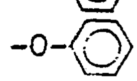 | 110-11 |
| 14 | 2,4-$Cl_2$ | N | CH(OH) | 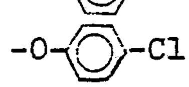 | 165(Zers.) (xHCl) |
| 15 | 2,4-$Cl_2$ | N | CH(OH) | 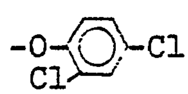 | 132 |
| 16 | 2,4-$Cl_2$ | N | CH(OH) | 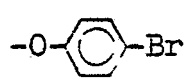 | 147(xHCl) |

| Bsp.Nr. | $Z_n$ | A | B | R | Schmelz-punkt(°C) |
|---|---|---|---|---|---|
| 17 | 4-Cl | N | CH(OH) | 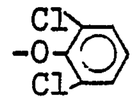 | 128-30 |
| 18 | 2,4-$Cl_2$ | CH | CO | 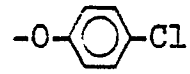 | 134-40(Zers.) (xHCl) |
| 19 | 2,4-$Cl_2$ | CH | CO | 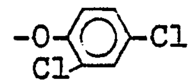 | 167-75 (xHCl) |
| 20 | 4-Cl | CH | CO | 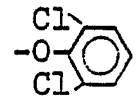 | 115(Zers.) (xHCl) |
| 21 | 2,4-$Cl_2$ | CH | CH(OH) | 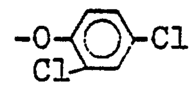 | 118 |
| 22 | 4-Cl | CH | CH(OH) | 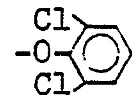 | 172 |
| 23 | 4-Cl | N. | CO | 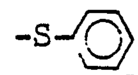 | 75-78 |
| 24 | 4-Cl | N | CO | 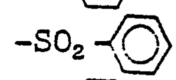 | 25-30 |
| 25 | 4-Cl | N | CH(OH) | 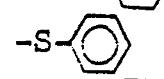 | 22-25 |
| 26 | 4-Cl | N | CH(OH) | 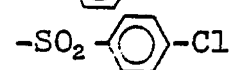 | 77-80 |
| 27 | 4-Cl | CH | CO | 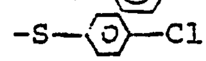 | $n_D^{20}$ = 1,5838 |
| 28 | 4-Cl | CH | CH(OH) | 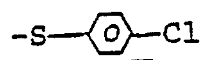 | 114-16 |
| 29 | 4-Cl | CH | CO | 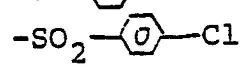 | 134-38 |
| 30 | 4-Cl | CH | CH(OH) | $-OCH_3$ | 112-20 |
| 31 | 2,4-Cl | CH | CO | 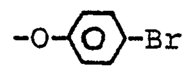 | 108-10 (x HCl) (Zers.) |
| 32 | 2,4-$Cl_2$ | CH | CH(OH) | 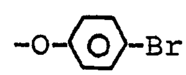 | 120-23 |
| 33 | 4-Cl | CH | CH(OH) | 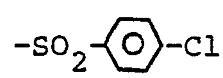 | 176-83 |
| 34 | 2,4-$Cl_2$ | CH | CH(OH) | 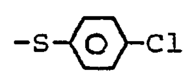 | 132 |
| 35 | 2,4-$Cl_2$ | N | CO | 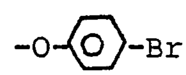 | 30 |
| 36 | 2,4-$Cl_2$ | CN | CO | 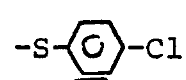 | Öl |
| 37 | 4-F | CH | CO | 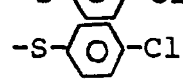 | Öl |
| 38 | 4-F | CH | CH(OH) | 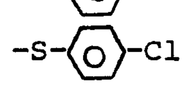 | 25 |

Verwendungsbeispiele

In den nachfolgenden Beispielen werden die nachsthend angegebenen Verbindungen als Vergleichssubstanzen eingesetzt:

(A) 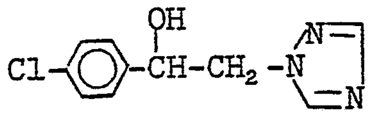

(B) 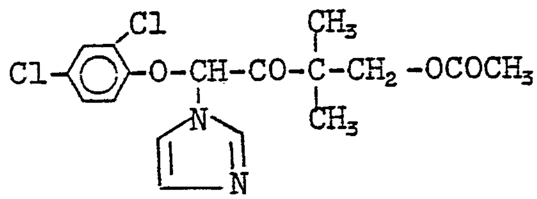

(C) 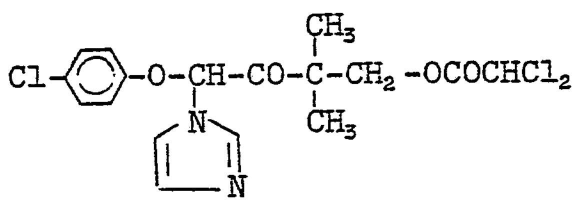

(D) 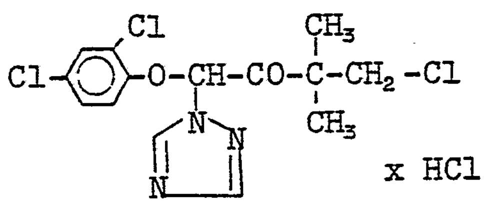
x HCl

(E) 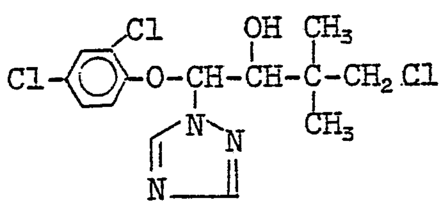

(F) 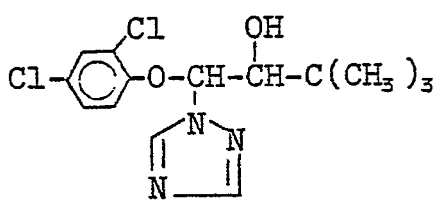

(G) 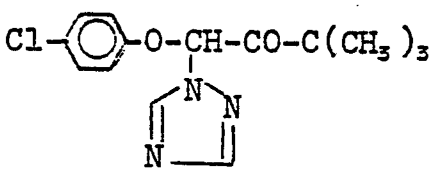

(H) 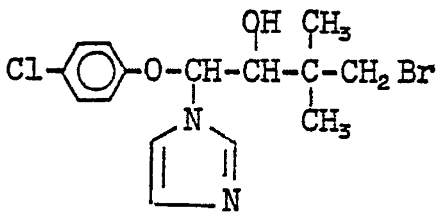

Beispiel A

Erysiphe-Test (Gerste) /protektiv/

Lösungsmittel: 100 Gewichtsteile Dimethylformamid
Emulgator: 0,25 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.
Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20°C und einer relativen Luftfeuchtigkeit von ca. 80% aufgestellt, um die Entwicklung von Mehltaupusteln zu begünstigen.

7 Tage nach der Inokulation erfolgt die Auswertung.

Eine deutliche Ueberlegenheit in der Wirksamkeit gegenüber den aus dem Stand der Technik bekannten Verbindungen (A) und (B) zeigen bei diesem Test z.B. die Verbindungen gemäß folgender Herstellungsbeispiele: 2,18,19,22,12,13,1, 14,7,8 und 9.



BAD ORIGINAL

Tabelle A

Erysiphe-Test (Gerste) / protektiv

| Wirkstoff | Wirkstoffkonzentration in der Spritzbrühe in Gew.-% | Krankheitsbefall in % der unbehandelten Kontrolle |
|---|---|---|
| 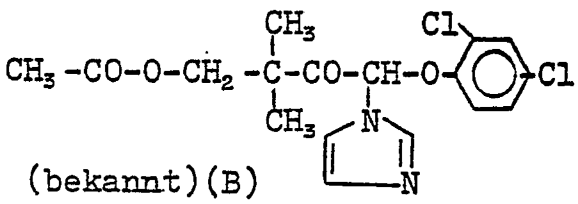 (bekannt) (B) | 0,025 | 50,o |
| 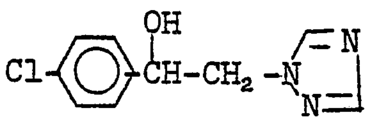 (bekannt) (A) | 0,025 | 100 |
| 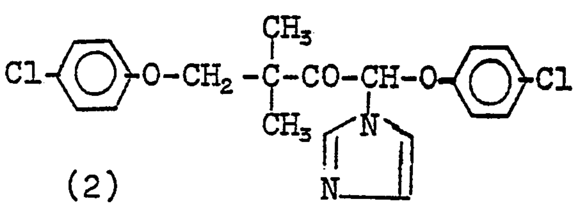 (2) | 0,025 | 16,3 |
| 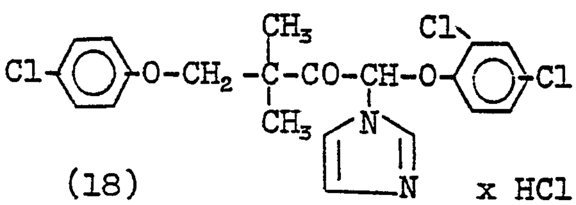 x HCl (18) | 0,025 | 11,3 |
| 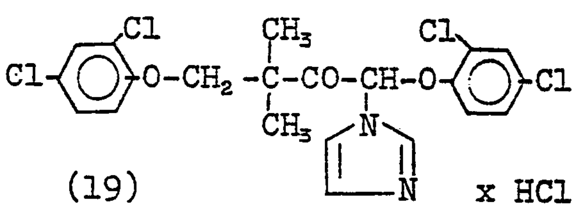 x HCl (19) | 0,025 | 3,8 |
| 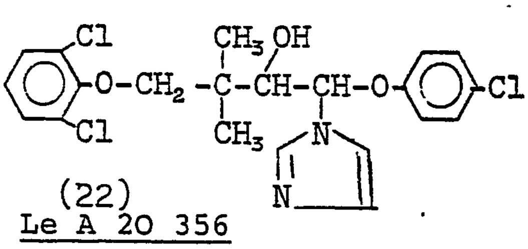 (22) | 0,025 | 0,0 |

Tabelle A (Fortsetzung)

Erysiphe-Test (Gerste) / protektiv

| Wirkstoff | Wirkstoffkonzentration in der Spritzbrühe in Gew.-% | Krankheitsbefall in % der unbehandelten Kontrolle |
|---|---|---|
| 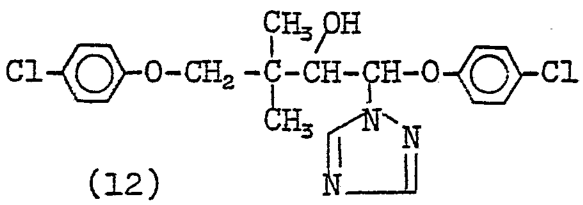 (12) | 0,025 | 33,8 |
| 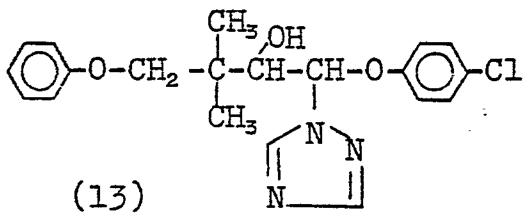 (13) | 0,025 | 8,8 |
| 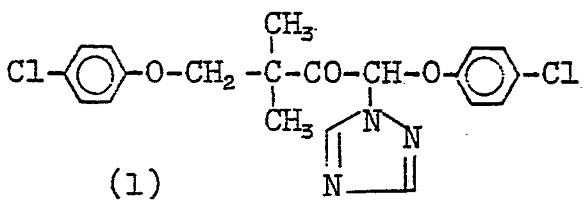 (1) | 0,025 | 11,3 |
| 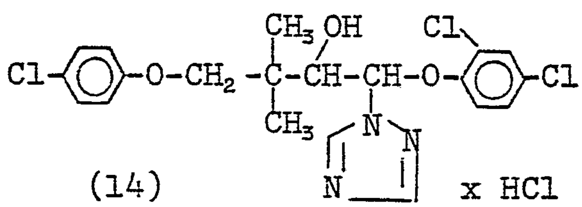 x HCl (14) | 0,025 | 16,3 |
| 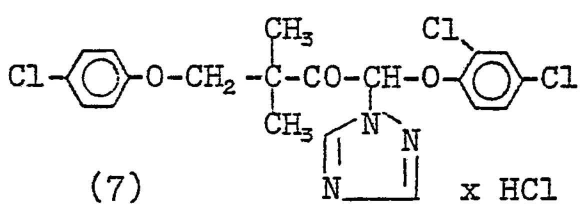 x HCl (7) | 0,025 | 3,8 |
| 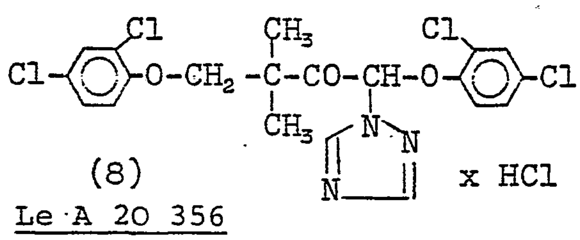 x HCl (8) | 0,025 | 7,5 |

Tabelle A(Fortsetzung)

Erysiphe-Test (Gerste) / protektiv

| Wirkstoff | Wirkstoffkonzentration in der Spritzbrühe in Gew.-% | Krankheitsbefall in % der unbehandelten Kontrolle |
|---|---|---|
| 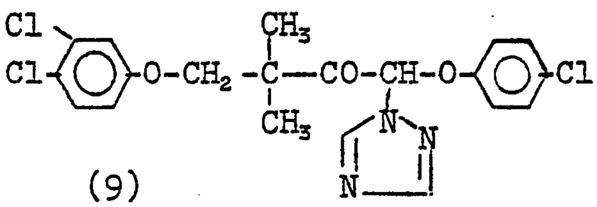 (9) | 0,025 | 0,0 |

Beispiel B

Uromyces-Test (Buschbohnen) / protektiv

Lösungsmittel: 4,7 Gewichtsteile Aceton

Emulgator: 0,3 Gewichtsteile Alkylarylpolyglykolether

Wasser: 95,0 Gewichtsteile

Man vermischt die für die gewünschte Wirkstoffkonzentration in der Spritzflüssigkeit notwendige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man die jungen Bohnenpflanzen, die sich im 2-Blattstadium befinden, bis zur Tropfnässe. Die Pflanzen verbleiben zum Abtrocknen 24 Stunden bei 20-22°C und einer relativen Luftfeuchtigkeit von 70% im Gewächshaus. Anschließend werden sie mit einer wässrigen Uredosporensuspension des Bohnenrosterregers (Uromyces phaseoli) inokuliert und 24 Stunden lang in einer dunklen Feuchtkammer bei 20-22°C und 100% relativer Luftfeuchtigkeit inkubiert.

Die Pflanzen werden dann unter intensiver Belichtung für 9 Tage bei 20-22°C und einer relativen Luftfeuchtigkeit von 70-80% im Gewächshaus aufgestellt.

10 Tage nach der Inokulation wird der Befall der Pflanzen bestimmt. Die erhaltenen Boniturwerte werden in Prozent Befall umgerechnet. 0% bedeutet keinen Befall, 100 % bedeutet, daß die Pflanzen vollständig befallen sind.

Eine deutliche Ueberlegenheit in der Wirksamkeit gegenüber den aus dem Stand der Technik bekannten Verbindungen (E),(F) und (G) zeigen bei diesem Test z.B. die Verbindungen gemäß folgender Herstellungsbeispiele: 12,1,14,7, 8,15 und 9.

Tabelle B

Uromyces-Test / protektiv

| Wirkstoff | Befall in % bei einer Wirkstoffkonzentration von: 0,001 % |
|---|---|
| 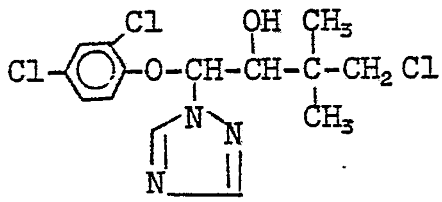 (bekannt) (E) | 71 |
| 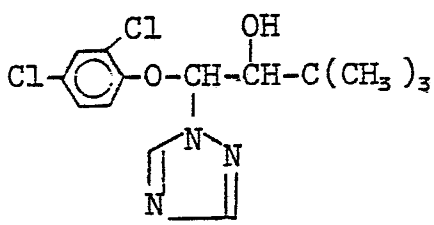 (bekannt) (F) | 75 |
| 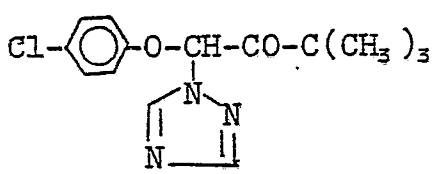 (bekannt) (G) | 62 |
| 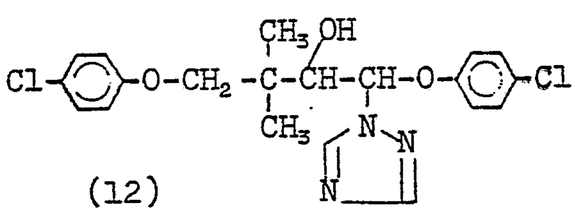 (12) | 10 |
| 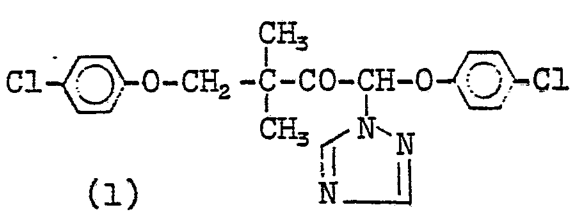 (1) | 0 |

**T a b e l l e B (Fortsetzung)**

Uromyces-Test / protektiv

| W i r k s t o f f | Befall in % bei einer Wirkstoffkonzentration von: 0,001 % |
|---|---|
| 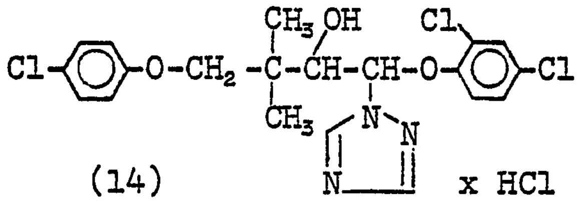 (14) x HCl | 29 |
| 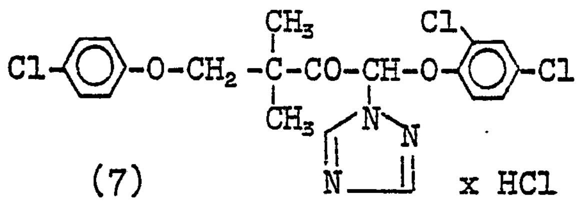 (7) x HCl | 29 |
| 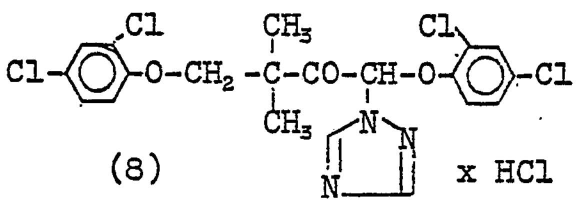 (8) x HCl | 50 |
| 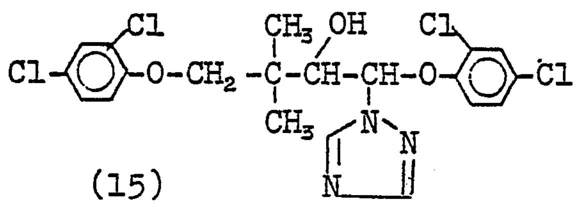 (15) | 50 |
| 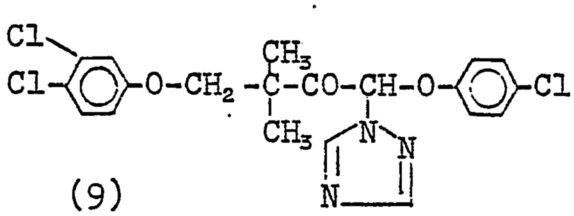 (9) | 21 |

Beispiel C

Fusicladium-Test (Apfel) / protektiv

Lösungsmittel: 4,7 Gewichtsteile Aceton
Emulgator : 0,3 Gewichtsteile Alkylarylpolyglykolether
Wasser : 95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.
Mit der Spritzflüssigkeit bespritzt man junge Apfelsämlinge, die sich im 4- bis 6-Blattstadium befinden, bis zur Tropfnässe. Die Pflanzen verbleiben 24 Stunden bei 20°C und einer relativen Luftfeuchtigkeit von 70% im Gewächshaus. Anschließend werden sie mit einer wässrigen Konidiensuspension des Apfelschorferregers (Fusicladium dendriticum) inokuliert und 18 Stunden lang in einer Feuchtkammer bei 18 bis 20°C und 100 % relativer Luftfeuchtigkeit inkubiert.

Die Pflanzen kommen dann erneut für 14 Tage ins Gewächshaus.

15 Tage nach der Inokulation wird der Befall der Sämlinge bestimmt. Die erhaltenen Boniturwerte werden in Prozent Befall umgerechnet. 0 % bedeutet keinen Befall, 100 % bedeutet, daß die Pflanzen vollständig befallen sind.

Eine deutliche Ueberlegenheit in der Wirksamkeit gegenüber der aus dem Stand der Technik bekannten Verbindung (D) zeigen bei diesem Test z.B. die Verbindungen gemäß folgender Herstellungsbeispiele: 12 und 13.

0042980



## Tabelle C

Fusicladium-Test(Apfel) / protektiv

| Wirkstoff | Befall in % bei einer Wirkstoffkonzentration von 0,0005 % |
|---|---|
| 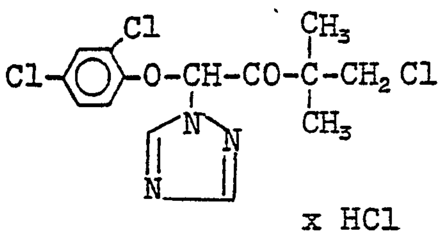 x HCl (bekannt) (D) | 15 |
| 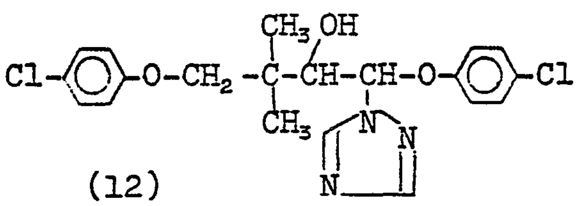 (12) | 10 |
| 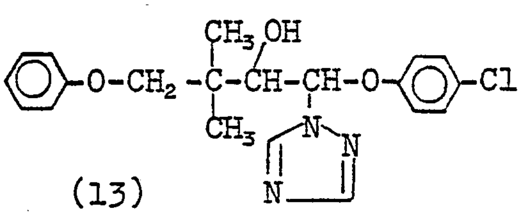 (13) | 4 |

Beispiel D

Erysiphe-Test (Gurken)/ Protektiv

Lösungsmittel: 4,7 Gewichtsteile Aceton

Emulgator: 0,3 Gewichtsteile Alkyl-aryl-polyglykolether

Wasser: 95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Gurkenpflanzen mit etwa drei Laubblättern bis zur Tropfnässe. Die Gurkenpflanzen verbleiben zur Trocknung 24 Stunden im Gewächshaus. Dann werden sie zur Inokulation mit Konidien des Pilzes Erysiphe cichoracearum bestäubt. Die Pflanzen werden anschließend bei 23 bis 24°C und bei einer relativen Luftfeuchtigkeit von ca 75 % im Gewächshaus aufgestellt.

Nach 12 Tagen wird der Befall der Gurkenpflanzen bestimmt. Die erhaltenen Boniturwerte werden in Prozent Befall umgerechnet. 0 % bedeutet keinen Befall, 100 % bedeutet, daß die Pflanzen vollständig befallen sind.

Eine deutliche Ueberlegenheit in der Wirksamkeit gegenüber den aus dem Stand der Technik bekannten Verbindungen (C) und (H) zeigen bei diesem Test z.B. die Verbindungen gemäß folgender Herstellungsbeispiele: 2,18,20 und 22.

## Tabelle D

Erysiphe-Test (Gurken) / Protektiv

| Wirkstoff | Befall in % bei einer Wirkstoffkonzentration von 0,0005 % |
|---|---|
| 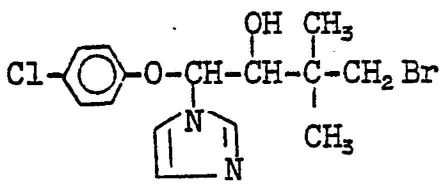 (bekannt) (H) | 75 |
| 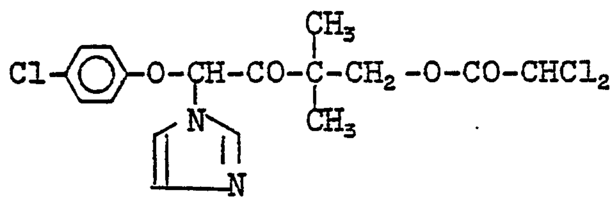 (bekannt) (C) | 100 |
| 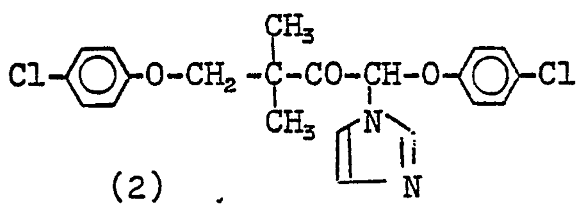 (2) | 12 |
| 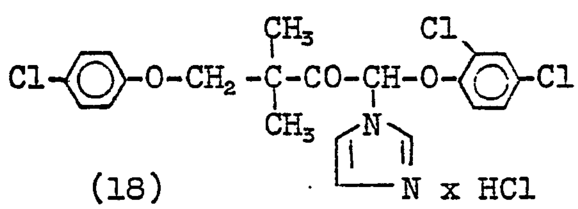 (18) x HCl | 19 |
| 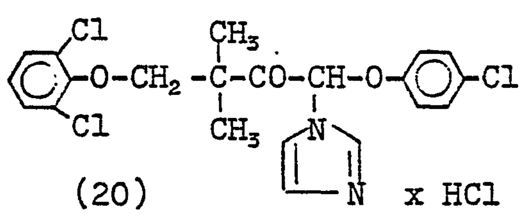 (20) x HCl | 19 |
| 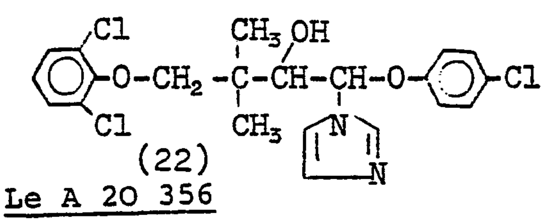 (22) | 50 |

Patentansprüche

1) 4-Substituierte 1-Azolyl-1-phenoxy-3,3-dimethyl-butan-2-one und -ole der allgemeinen Formel

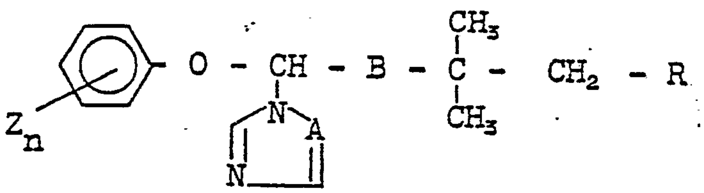

(I)

in welcher

A für ein Stickstoffatom oder die CH-Gruppe steht,

B für die Ketogruppe oder die CH(OH)-Gruppe steht,

R für die Gruppierung $X-R^1$ oder Cyano steht, wobei

$R^1$ für Alkyl, gegebenenfalls substituiertes Aryl oder gegebenenfalls substituiertes Aralkyl steht, und

X für Sauerstoff, Schwefel, die SO- oder $SO_2$-Gruppe steht,

Z für Halogen, Alkyl, Halogenalkyl, Alkoxy, Alkylthio, Alkylsulfonyl, Nitro, Cyano, Alkoxycarbonyl oder gegebenenfalls substituiertes Phenyl steht und

n für 0, 1, 2 oder 3 steht,

sowie deren physiologisch verträglichen Säureadditions-Salze und Metallsalz-Komplexe.

2) Verfahren zur Herstellung von 4-substituierten 1-Azolyl-1-phenoxy-3,3-dimethyl-butan-2-onen und -olen gemäß Formel I in Anspruch 1, dadurch gekennzeichnet, daß man Halogenetherketone der Formel

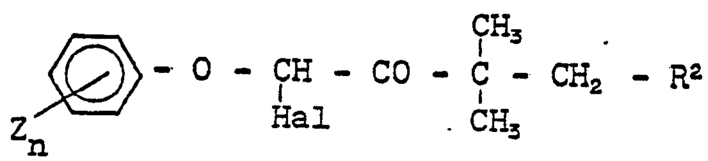

(II)

in welcher

| | |
|---|---|
| Z und n | die in Anspruch 1 angegebene Bedeutung haben, |
| Hal | für Halogen, vorzugsweise Chlor oder Brom steht und |
| $R^2$ | für $X^1$-$R^1$ oder Cyano steht, wobei |
| $X^1$ | für Sauerstoff oder Schwefel steht und |
| $R^1$ | die in Anspruch 1 angegebene Bedeutung hat, |

mit Azolen der Formel

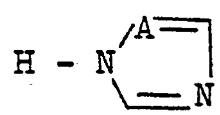

(III)

in welcher

A die oben angegebene Bedeutung hat,

gegebenenfalls in Gegenwart eines Verdünnungsmittels und in Gegenwart eines Säurebindemittels umsetzt, und gegebenenfalls noch die erhaltenen Keto-Derivate der Formel

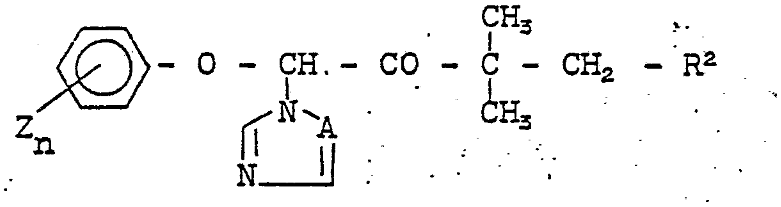 (Ia)

in welcher

A, $R^2$, Z und n die oben angegebene Bedeutung haben,

nach bekannten Methoden in üblicher Weise reduziert, und gegebenenfalls noch die erhaltenen Thio-Derivate der Formel

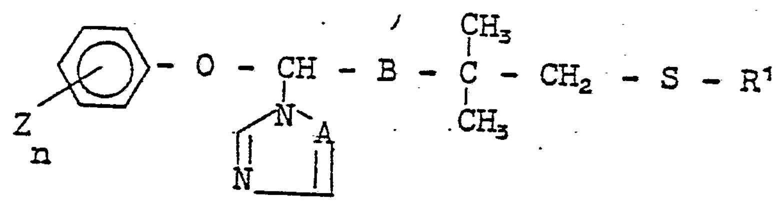 (Ib)

in welcher

A, B, Z, $R^1$ und n die oben angegebene Bedeutung haben,

nach bekannten Methoden in üblicher Weise oxidiert, und endlich noch gegebenenfalls an die so erhaltenen Verbindungen der Formel (I) anschließend eine Säure oder ein Metallsalz addiert.

3) Fungizide Mittel, gekennzeichnet durch einen Gehalt an mindestens einem 4-substituierten 1-Azolyl-1-phenoxy-3,3-dimethyl-butan-2-on oder -ol der Formel I.

4) Verfahren zur Bekämpfung von Pilzen, dadurch gekennzeichnet, daß man 4-substituierte 1-Azolyl-1-phenoxy-3,3-dimethyl-butan-2-one oder -ole der Formel I auf Pilze oder ihren Lebensraum einwirken läßt.

5) Verwendung von 4-substituierten 1-Azolyl-1-phenoxy-3,3-dimethyl-butan-2-onen oder -olen der Formel I zur Bekämpfung von Pilzen.

6) Verfahren zur Herstellung von fungiziden Mitteln, dadurch gekennzeichnet, daß man 4-substituierte 1-Azolyl-1-phenoxy-3,3-dimethyl-butan-2-one oder -ole der Formel I mit Streckmitteln und/oder oberflächenaktiven Mitteln vermischt.